# EUROPÄISCHE PATENTSCHRIFT

(11) **EP 2 139 998 B1**
(45) Veröffentlichungstag und Bekanntmachung des Hinweises auf die Patenterteilung: **12.03.2014**
(21) Anmeldenummer: 08734708.4
(22) Anmeldetag: 20.03.2008
(51) Int. Cl.: C12N 9/90, C12P 7/10, C12N 15/81

(54) **VEKTOR MIT CODON-OPTIMIERTEN GENEN EINES ARABINOSE-STOFFWECHSELWEGES ZUR ARABINOSE-UMSETZUNG IN HEFE ZUR ETHANOLHERSTELLUNG**
VECTOR WITH CODON-OPTIMISED GENES FOR AN ARABINOSE METABOLIC PATHWAY FOR ARABINOSE CONVERSION IN YEAST FOR ETHANOL PRODUCTION
VECTEUR À GÈNES OPTIMISÉS AU CODON D'UNE VOIE MÉTABOLIQUE DE L'ARABINOSE, EN VUE DE LA TRANSFORMATION DE L'ARABINOSE DANS DES LEVURES POUR LA PRODUCTION D'ÉTHANOL

(30) Priorität: 05.04.2007 DE 102007016534
(43) Veröffentlichungstag der Anmeldung: 06.01.2010
(73) Patentinhaber: Butalco GmbH, 6363 Fürigen (CH)
(72) Erfinder: BOLES, Eckhard, 63303 Dreieich (DE); ROTHER, Beate, 37574 Einbeck (DE)
(74) Vertreter: Engelhard, Markus
(86) Internationale Anmeldenummer: PCT/EP2008/002277
(87) Internationale Veröffentlichungsnummer: WO 2008/122354

(56) Entgegenhaltungen:
- WO-A-98/50524
- WO-A-03/008593
- WO-A-2006/096130
- SEDLAK M ET AL: "EXPRESSION OF E. COLI ARABAD OPERON ENCODING ENZYMES FOR METABOLIZING L-ARABINOSE IN SACCHAROMYCES CEREVISIAE" ENZYME AND MICROBIAL TECHNOLOGY, STONEHAM, MA, US, Bd. 28, 1. Januar 2001 (2001-01-01), Seiten 16-24, XP002950458 ISSN: 0141-0229 in der Anmeldung erwähnt
- DATABASE NCBI 29. September 2004 (2004-09-29), "AraA, Bacillus licheniformis" XP002487029 Database accession no. AAU41894
- BENNETZEN J L ET AL: "CODON SELECTION IN YEAST SACCHAROMYCES-CEREVISIAE" JOURNAL OF BIOLOGICAL CHEMISTRY, Bd. 257, Nr. 6, 1982, Seiten 3026-3031, XP009102736 ISSN: 0021-9258 in der Anmeldung erwähnt
- FONSECA C ET AL: "L-Arabinose transport and catabolism in yeast" FEBS JOURNAL, BLACKWELL PUBLISHING, LONDON, GB, Bd. 274, 1. Januar 2007 (2007-01-01), Seiten 3589-3600, XP007903587 ISSN: 1742-464X
- VEITH B ET AL: "THE COMPLETE GENOME SEQUENCE OF BACILLUS LICHENIFORMIS DSM13, AN ORGANISM WITH GREAT INDUSTRIAL POTENTIAL", JOURNAL OF MOLECULAR MICROBIOLOGY AND BIOTECHNOLOGY, HORIZON SCIENTIFIC PRESS, WYMONDHAM, GB, vol. 7, no. 4, 1 September 2004 (2004-09-01), pages 204-211, XP009047713, ISSN: 1464-1801, DOI: 10.1159/000079829
- NOELLING J ET AL: "GENOME SEQUENCE AND COMPARATIVE ANALYSIS OF THE SOLVENT-PRODUCING BACTERIUM CLOSTRIDIUM ACETOBUTYLICUM", JOURNAL OF BACTERIOLOGY, AMERICAN SOCIETY FOR MICROBIOLOGY, WASHINGTON, DC; US, vol. 183, no. 16, 1 August 2001 (2001-08-01), pages 4823-4838, XP002950775, ISSN: 0021-9193, DOI: 10.1128/JB.183.16.4823-4838.2001
- WIEDEMANN BEATE ET AL: "Codon-optimized bacterial genes improve L-Arabinose fermentation in recombinant Saccharomyces cerevisiae.", APPLIED AND ENVIRONMENTAL MICROBIOLOGY APR 2008, vol. 74, no. 7, April 2008 (2008-04), pages 2043-2050, ISSN: 1098-5336

### SEQUENCE LISTING

<110> Johann Wolfgang Goethe-Universität
<120> Neuer Expressionsvektor von Codon-optimierten Genen eines bakteriellen Arabinose-Stoffwechselweges zur Arabinose-Umsetzung in Hefe
<130> U30171PCT
<160> 7
<170> PatentIn version 3.2
<210> 1
   <211> 1704
   <212> DNA
   <213> Artificial
<220>
   <223> Gensequenz des offenen Leserahmens (ORF) von araBmut aus E. coli in einer Codon-optimierten Form
<400> 1
   atggctattg ctattggttt ggacttcggt tctgactctg ttagagcttt ggctgttgac      60
   tgtgcttctg gtgaagaaat tgctacctct gttgaatggt acccaagatg gcaaaagggt     120
   caattctgtg acgctccaaa caaccaattc agacaccacc caagagacta cattgaatct     180
   atggaagctg ctttgaagac cgttttggct gaattgtctg ttgaacaaag agctgctgtt     240
   gttggtattg gtgttgactc taccggttct accccagctc caattgacgc tgacggtaac     300
   gttttggctt tgagaccaga attcgctgaa aacccaaacg ctatgttcgt tttgtggaag     360
   aaccacaccg ctgttgaaga agctgaagaa attaccagat tgtgtcacgc tccaggtaac     420
   gttgactact ctagatacat tggtggtatt tactcttctg aatggttctg ggctaagatt     480
   ttgcacgtta ccagacaaga ctctgctgtt gctcaatctg ctgcttcttg gattgaattg     540
   tgtgactggg ttccagcttt gttgtctggt accaccagac cacaagacat tagaagaggt     600
   agatgttctg ctggtcacaa gtctttgtgg cacgaatctt ggggtggttt gccaccagct     660
   tctttcttcg acgaattgga cccaattttg aacagacact tgccatctcc attgttcacc     720
   gacacctgga ccgctgacat tccagttggt accttgtgtc cagaatgggc tcaaagattg     780
   ggtttgccag aatctgttgt tatttctggt ggtgctttcg actgtcacat gggtgctgtt     840
   ggtgctggtg ctcaaccaaa cgctttggtt aaggttattg gtacctctac ctgtgacatt     900
   ttgattgctg acaagcaatc tgttggtgaa agagctgtta agggtatttg tggtcaagtt     960
   gacggttctg ttgttccagg tttcattggt ttggaagctg gtcaatctgc tttcggtgac    1020
   atttacgctt ggttcggtag agttttgtct tggccattgg aacaattggc tgctcaacac    1080
   ccagaattga aggctcaaat taacgcttct caaaagcaat tgttgccagc tttgaccgaa    1140
   gcttgggcta agaacccatc tttggaccac ttgccagttg ttttggactg gttcaacggt    1200
   agaagatctc caaacgctaa ccaaagattg aagggtgtta ttaccgactt gaacttggct    1260
   accgacgctc cattgttgtt cggtggtttg attgctgcta ccgctttcgg tgctagagct    1320
   attatggaat gtttcaccga ccaaggtatt gctgtcaaca acgttatggc tttgggtggt    1380
   attgctagaa agaaccaagt tattatgcaa gcttgttgtg acgttttgaa cagaccattg    1440
   caaattgttg cttctgacca atgttgtgct ttgggtgctg ctattttcgc tgctgttgct    1500
   gctaaggttc acgctgacat tccatctgct caacaaaaga tggcttctgc tgttgaaaag    1560
   accttgcaac caagatctga acaagctcaa agattcgaac aattgtacag aagataccaa    1620
   caatgggcta tgtctgctga acaacactac ttgccaacct ctgctccagc tcaagctgct    1680
   caagctgttg ctaccttgta ataa                                           1704
<210> 2
   <211> 699
   <212> DNA
   <213> Artificial
<220>
   <223> Gensequenz des offenen Leserahmens (ORF) von araD aus E. coli in einer Codon-optimierten Form
<400> 2
   atgttggaag acttgaagag acaagttttg gaagctaact tggctttgcc aaagcacaac     60
   ttggttacct tgacctgggg taacgtttct gctgttgaca gagaaagagg tgttttcgtt    120
   attaagccat ctggtgttga ctactctgtt atgaccgctg acgacatggt tgttgtttct    180
   attgaaaccg gtgaagttgt tgaaggtacc aagaagccat cttctgacac cccaacccac    240
   agattgttgt accaagcttt cccatctatt ggtggtattg ttcacaccca ctctagacac    300
   gctaccattt gggctcaagc tggtcaatct attccagcta ccggtaccac ccacgctgac    360
   tacttctacg gtaccattcc atgtaccaga aagatgaccg acgctgaaat taacggtgaa    420
   tacgaatggg aaaccggtaa cgttattgtt gaaaccttcg aaaagcaagg tattgacgct    480
   gctcaaatgc caggtgtttt ggttcactct cacggtccat tcgcttgggg taagaacgct    540
   gaagacgctg ttcacaacgc tattgttttg gaagaagttg cttacatggg tattttctgt    600
   agacaattgg ctccacaatt gccagacatg caacaaacct tgttggacaa gcactacttg    660
   agaaagcacg gtgctaaggc ttactacggt caataataa                           699
<210> 3
   <211> 1485
   <212> DNA
   <213> Artificial
<220>
   <223> Gensequenz des offenen Leserahmens (ORF) von araA aus Bacillus licheniformis in einer Codon-optimierten Form
<400> 3
   atgattcaag ctaagaccca cgttttctgg ttcgttaccg gttctcaaca cttgtacggt 60
   gaagaagctg ttcaagaagt tgaagaacac tctaagatga tttgtaacgg tttgaacgac 120
   ggtgacttga gattccaagt tgaatacaag gctgttgcta cctctttgga cggtgttaga 180
   aagttgttcg aagaagctaa cagagacgaa gaatgtgctg gtattattac ctggatgcac 240
   accttctctc cagctaagat gtggattcca ggtttgtctg aattgaacaa gccattgttg 300
   cacttccaca cccaattcaa cagagacatt ccatgggaca agattgacat ggacttcatg 360
   aacattaacc aatctgctca cggtgacaga gaatacggtt tcattggtgc tagattgggt 420
   attccaagaa aggttattgc tggttactgg gaagacagag aagttaagag atctattgac 480
   aagtggatgt ctgctgctgt tgcttacatt gaatctagac acattaaggt tgctagattc 540
   ggtgacaaca tgagaaacgt tgctgttacc gaaggtgaca agattgaagc tcaaattcaa 600
   ttgggttggt ctgttgacgg ttacggtatt ggtgacttgg ttaccgaaat taacgctgtt 660
   tctgaacaat ctttgtctga attgatttct gaatacgaag aattgtacga atggccagaa 720
   ggtgaagctg ctagagaatc tgttaaggaa caagctagaa ttgaattggg tttgaagaga 780
   ttcttgtctt ctggtggtta caccgctttc accaccacct tcgaagactt gcacggtatg 840
   aagcaattgc caggtttggc tgttcaaaga ttgatggctg aaggttacgg tttcggtggt 900
   gaaggtgact ggaagaccgc tgctttggtt agaatgatga agatgatggc tggtggtaag 960
   gaaacctctt tcatggaaga ctacacctac cacttcgaac caggtaacga aatgattttg 1020
   ggttctcaca tgttggaagt ttgtccatct attgctgaac acaagccaag aattgaagtt 1080
   cacccattgt ctatgggtgc taaggacgac ccagctagat tggttttcga cggtattgct 1140
   ggtccagctg tcaacgtttc tttgattgac ttgggtggta gattcagatt ggttattaac 1200
   aaggttgaag ctgttaaggt tccacacgac atgccaaact tgccagttgc tagagttttg 1260
   tggaagccac aaccatcttt gagaacctct gctgaagctt ggattttggc tggtggtgct 1320
   caccacacct gtttgtctta ccaattgacc gctgaacaaa tgttggactg ggctgaaatg 1380
   tctggtattg aagctgtttt gattaacaga gacaccacca ttttgaactt gagaaacgaa 1440
   ttgaagtggt ctgaagctgc ttacagattg agaaagttct aataa 1485
<210> 4
   <211> 1482
   <212> DNA
   <213> Bacillus licheniformis
<400> 4
   atgattcagg caaagacgca tgtgttttgg tttgtgacag gcagccagca tttatatggc 60
   gaagaggcgg tacaagaggt agaagagcat tccaaaatga tctgcaacgg attaaatgac 120
   ggagatttaa ggtttcaagt cgagtacaaa gcggtggcca cttcgctgga cggcgtcaga 180
   aaactgtttg aagaggcgaa ccgggacgaa gagtgcgcag gcatcatcac ctggatgcat 240
   acgttttcac cggccaaaat gtggattccc ggcctttccg agctgaataa gccgctgctc 300
   cattttcata cccagtttaa ccgggacatt ccgtgggata aaatcgacat ggatttcatg 360
   aatattaatc agtctgccca cggcgaccgc gaatacggtt ttatcggagc gagattgggc 420
   attcctcgaa aagtaatcgc cggatattgg gaagacagag aagtaaagcg ctcgatcgac 480
   aaatggatga gcgcagcggt cgcatatatt gaaagccgcc atatcaaagt cgcccgattt 540
   ggggacaaca tgcggaatgt ggcggtaaca gaaggagata agattgaagc gcagattcag 600
   cttggctggt ctgtcgacgg atatggaatc ggcgatctcg tcacggagat caatgctgta 660
   tcagaacaga gtctaagcga gctcatctcc gaatatgagg agctttatga gtggccggaa 720
   ggcgaagcgg caagggaatc cgtcaaggag caggcgcgga ttgagcttgg attaaagcgc 780
   tttctttcga gcggaggcta tacggccttt acgacaacct ttgaagacct ccacggcatg 840
   aagcagcttc cgggtttggc tgttcagcgg ctgatggctg aaggctatgg ctttggagga 900
   gaaggagatt ggaaaacggc tgcgcttgtc aggatgatga aaatgatggc cggcggaaaa 960
   gaaacgtcat ttatggagga ttacacgtac cattttgaac cgggcaatga aatgatcctc 1020
   ggctcccaca tgcttgaagt gtgcccttcg attgccgaac acaaaccgag aattgaagtc 1080
   catccgctgt caatgggtgc aaaggatgat ccggcgcggc tcgtgtttga cggaattgca 1140
   ggtcctgcgg tcaatgtctc ccttattgat ttgggcggac gtttcaggct tgtcatcaat 1200
   aaagtggaag ctgtgaaggt tccgcatgat atgccgaatc ttccggtggc gcgcgtactt 1260
   tggaagcctc agccgtctct gagaacatcg gctgaagcgt ggattttggc tggcggtgct 1320
   caccatacat gcctttcgta ccagctgaca gccgagcaga tgctcgattg ggccgaaatg 1380
   tcgggcattg aagcggtgtt gatcaatcgt gatacaacga ttttaaatct tcgcaatgaa 1440
   ctcaaatgga gtgaagctgc atatcggctt cgcaagtttt aa 1482
<210> 5
   <211> 1467
   <212> DNA
   <213> Clostridium acetobutylicum
<400> 5
   atgttagaaa ataaaaagat ggaattttgg tttgtagtag gaagtcaaca tttatatggt 60
   gaagaggctt taaaagaagt aagaaaaaat tctgagacaa ttgtagatga attaaataaa 120
   agtgctaatc ttccatataa aataatattt aaagatttag caacttctgc tgataaaata 180
   aaggaaataa tgaaggaagt taactataga gatgaagtag caggagttat aacttggatg 240
   catacgtttt ctccagctaa aatgtggata gcaggtacaa agatattaca aaaaccttta 300
   cttcattttg caactcaata taatgaaaat attccatgga aaacaataga tatggattat 360
   atgaacttac atcaaagtgc tcatggagat agagagtatg gatttattaa tgcaagactt 420
   aaaaagcata ataaagttgt tgtaggatat tggaaggata aagaagttca aaaacaagtt 480
   tcagattgga tgaaggttgc tgcaggatat attgcaagtg aaagcataaa agttgcacgt 540
   tttggtgata acatgcgtaa tgttgcagtt acagagggag ataaagtaga agctcaaata 600
   caattcggat ggacagtaga ttactttggt ataggtgatt tagttgctga aatggacaag 660
   gtaagccaag atgaaataaa taaaacttat gaagaattta aagatttata tattttagat 720
   ccaggtgaaa atgatcctgc tttctatgag aaacaagtta aagaacaaat caaaattgaa 780
   ataggattaa gaaggttctt agaaaaagga aattataatg catttacaac aaactttgaa 840
   gatctttatg gaatgaaaca gttacctgga cttgcagtac aacgtttaaa tgctgaaggc 900
   tatggctttg caggcgaagg agactggaaa actgcagctt tagatagatt attaaaggtt    960
   atgactaata atactgctac aggttttatg gaagattaca catatgaact tagtcgtgga   1020
   aatgagaagg cattaggagc tcatatgctt gaagttgacc caacttttgc ttcagataaa 1080
   ccaaaggtta ttgttaaacc actaggaatt ggagataaag aagatccagc acgtttaatc 1140
   tttaatggtt caacaggaaa aggtgtagca gtttcaatgc ttgatttagg aacacattat 1200
   cgtttaataa taaacggact tacagcagtg aaaccagatg aagacatgcc aaacctacca 1260
   gttgctaaaa tggtatggaa accagaacca aacttcattg aaggagttaa atcttggatt 1320
   tatgcaggtg gcggacatca tacagtggtt tcactagaat taacagtaga acaggtttat 1380
   gattggagtc gtatggtagg cttggaagct gtaataatag ataaggatac taaattaaga 1440
   gatataatag aaaagacaac aaaataa 1467
<210> 6
   <211> 493
   <212> PRT
   <213> Bacillus licheniformis
<400> 6
   Met Ile Gln Ala Lys Thr His Val Phe Trp Phe Val Thr Gly Ser Gln
   1               5                   10                  15
   His Leu Tyr Gly Glu Glu Ala Val Gln Glu Val Glu Glu His Ser Lys
               20                  25                  30
   Met Ile Cys Asn Gly Leu Asn Asp Gly Asp Leu Arg Phe Gln Val Glu
           35                  40                  45
   Tyr Lys Ala Val Ala Thr Ser Leu Asp Gly Val Arg Lys Leu Phe Glu
       50                  55                  60
   Glu Ala Asn Arg Asp Glu Glu Cys Ala Gly Ile Ile Thr Trp Met His
   65 70 75 80
   Thr Phe Ser Pro Ala Lys Met Trp Ile Pro Gly Leu Ser Glu Leu Asn
   85 90 95
   Lys Pro Leu Leu His Phe His Thr Gln Phe Asn Arg Asp Ile Pro Trp
   100 105 110
   Asp Lys Ile Asp Met Asp Phe Met Asn Ile Asn Gln Ser Ala His Gly
   115 120 125
   Asp Arg Glu Tyr Gly Phe Ile Gly Ala Arg Leu Gly Ile Pro Arg Lys
   130 135 140
   Val Ile Ala Gly Tyr Trp Glu Asp Arg Glu Val Lys Arg Ser Ile Asp
   145 150 155 160
   Lys Trp Met Ser Ala Ala Val Ala Tyr Ile Glu Ser Arg His Ile Lys
   165 170 175
   Val Ala Arg Phe Gly Asp Asn Met Arg Asn Val Ala Val Thr Glu Gly
   180 185 190
   Asp Lys Ile Glu Ala Gln Ile Gln Leu Gly Trp Ser Val Asp Gly Tyr
   195 200 205
   Gly Ile Gly Asp Leu Val Thr Glu Ile Asn Ala Val Ser Glu Gln Ser
   210 215 220
   Leu Ser Glu Leu Ile Ser Glu Tyr Glu Glu Leu Tyr Glu Trp Pro Glu
   225 230 235 240
   Gly Glu Ala Ala Arg Glu Ser Val Lys Glu Gln Ala Arg Ile Glu Leu
   245 250 255
   Gly Leu Lys Arg Phe Leu Ser Ser Gly Gly Tyr Thr Ala Phe Thr Thr
   260 265 270
   Thr Phe Glu Asp Leu His Gly Met Lys Gln Leu Pro Gly Leu Ala Val
   275 280 285
   Gln Arg Leu Met Ala Glu Gly Tyr Gly Phe Gly Gly Glu Gly Asp Trp
   290 295 300
   Lys Thr Ala Ala Leu Val Arg Met Met Lys Met Met Ala Gly Gly Lys
   305 310 315 320
   Glu Thr Ser Phe Met Glu Asp Tyr Thr Tyr His Phe Glu Pro Gly Asn
   325 330 335
   Glu Met Ile Leu Gly Ser His Met Leu Glu Val Cys Pro Ser Ile Ala
   340 345 350
   Glu His Lys Pro Arg Ile Glu Val His Pro Leu Ser Met Gly Ala Lys
   355 360 365
   Asp Asp Pro Ala Arg Leu Val Phe Asp Gly Ile Ala Gly Pro Ala Val
   370 375 380
   Asn Val Ser Leu Ile Asp Leu Gly Gly Arg Phe Arg Leu Val Ile Asn
   385 390 395 400
   Lys Val Glu Ala Val Lys Val Pro His Asp Met Pro Asn Leu Pro Val
   405 410 415
   Ala Arg Val Leu Trp Lys Pro Gln Pro Ser Leu Arg Thr Ser Ala Glu
   420 425 430
   Ala Trp Ile Leu Ala Gly Gly Ala His His Thr Cys Leu Ser Tyr Gln
   435 440 445
   Leu Thr Ala Glu Gln Met Leu Asp Trp Ala Glu Met Ser Gly Ile Glu
   450 455 460
   Ala Val Leu Ile Asn Arg Asp Thr Thr Ile Leu Asn Leu Arg Asn Glu
   465 470 475 480
   Leu Lys Trp Ser Glu Ala Ala Tyr Arg Leu Arg Lys Phe
   485 490
<210> 7
   <211> 488
   <212> PRT
   <213> Clostridium acetobutylicum
<400> 7
   Met Leu Glu Asn Lys Lys Met Glu Phe Trp Phe Val Val Gly Ser Gln
   1               5                   10                  15
   His Leu Tyr Gly Glu Glu Ala Leu Lys Glu Val Arg Lys Asn Ser Glu
               20                  25                  30
   Thr Ile Val Asp Glu Leu Asn Lys Ser Ala Asn Leu Pro Tyr Lys Ile
               35                  40                  45
   Ile Phe Lys Asp Leu Ala Thr Ser Ala Asp Lys Ile Lys Glu Ile Met
       50                  55                  60
   Lys Glu Val Asn Tyr Arg Asp Glu Val Ala Gly Val Ile Thr Trp Met
   65                  70                  75                  80
   His Thr Phe Ser Pro Ala Lys Met Trp Ile Ala Gly Thr Lys Ile Leu
                   85                  90                  95
   Gln Lys Pro Leu Leu His Phe Ala Thr Gln Tyr Asn Glu Asn Ile Pro
               100                 105                 110
   Trp Lys Thr Ile Asp Met Asp Tyr Met Asn Leu His Gln Ser Ala His
           115                 120                 125
   Gly Asp Arg Glu Tyr Gly Phe Ile Asn Ala Arg Leu Lys Lys His Asn
       130                 135                 140
   Lys Val Val Val Gly Tyr Trp Lys Asp Lys Glu Val Gln Lys Gln Val
   145                 150                 155                 160
   Ser Asp Trp Met Lys Val Ala Ala Gly Tyr Ile Ala Ser Glu Ser Ile
                   165                 170                 175
   Lys Val Ala Arg Phe Gly Asp Asn Met Arg Asn Val Ala Val Thr Glu
               180                 185                 190
   Gly Asp Lys Val Glu Ala Gln Ile Gln Phe Gly Trp Thr Val Asp Tyr
           195                 200                 205
   Phe Gly Ile Gly Asp Leu Val Ala Glu Met Asp Lys Val Ser Gln Asp
       210                 215                 220
   Glu Ile Asn Lys Thr Tyr Glu Glu Phe Lys Asp Leu Tyr Ile Leu Asp
   225                 230                 235                 240
   Pro Gly Glu Asn Asp Pro Ala Phe Tyr Glu Lys Gln Val Lys Glu Gln
                   245                 250                 255
   Ile Lys Ile Glu Ile Gly Leu Arg Arg Phe Leu Glu Lys Gly Asn Tyr
               260                 265                 270
   Asn Ala Phe Thr Thr Asn Phe Glu Asp Leu Tyr Gly Met Lys Gln Leu
           275                 280                 285
   Pro Gly Leu Ala Val Gln Arg Leu Asn Ala Glu Gly Tyr Gly Phe Ala
       290                 295                 300
   Gly Glu Gly Asp Trp Lys Thr Ala Ala Leu Asp Arg Leu Leu Lys Val
   305                 310                 315                 320
   Met Thr Asn Asn Thr Ala Thr Gly Phe Met Glu Asp Tyr Thr Tyr Glu
                   325                 330                 335
   Leu Ser Arg Gly Asn Glu Lys Ala Leu Gly Ala His Met Leu Glu Val
               340                 345                 350
   Asp Pro Thr Phe Ala Ser Asp Lys Pro Lys Val Ile Val Lys Pro Leu
           355                 360                 365
   Gly Ile Gly Asp Lys Glu Asp Pro Ala Arg Leu Ile Phe Asn Gly Ser
       370                 375                 380
   Thr Gly Lys Gly Val Ala Val Ser Met Leu Asp Leu Gly Thr His Tyr
   385                 390                 395                 400
   Arg Leu Ile Ile Asn Gly Leu Thr Ala Val Lys Pro Asp Glu Asp Met
                   405                 410                 415
   Pro Asn Leu Pro Val Ala Lys Met Val Trp Lys Pro Glu Pro Asn Phe
               420                 425                 430
   Ile Glu Gly Val Lys Ser Trp Ile Tyr Ala Gly Gly Gly His His Thr
           435                 440                 445
   Val Val Ser Leu Glu Leu Thr Val Glu Gln Val Tyr Asp Trp Ser Arg
       450                 455                 460
   Met Val Gly Leu Glu Ala Val Ile Ile Asp Lys Asp Thr Lys Leu Arg
   465                 470                 475                 480
   Asp Ile Ile Glu Lys Thr Thr Lys
                   485

## Beschreibung

### 1. Zusammenfassung

Die vorliegende Erfindung bezieht sich auf Wirtszellen enthaltend neue Expressionskassetten und Expressionsvektoren, die drei Nukleinsäuresequenzen für araA, araB und araD umfassen, die jeweils für ein Polypeptid eines L-Arabinose-Stoffwechselweges, kodieren. Die Erfindung bezieht sich insbesondere auf Wirtszellen enthaltend Expressionskassetten und Expressionsvektoren, die Codon-optimierte Nukleinsäuresequenzen für araA, araB und araD umfassen. Die Wirtszellen sind insbesondere modifizierte Hefe-Stämme die die Polypeptide des bakteriellen L-Arabinose-Stoffwechselweges exprimieren. Bei der Verwendung dieser modifizierten Wirtszellen wird Arabinose durch diese Zellen effektiver fermentiert, insbesondere zu Ethanol. Die vorliegende Erfindung ist somit unter anderem bedeutsam im Zusammenhang mit der Produktion von Biochemikalien aus Biomasse, wie z.B. Bioethanol.

### 2. Hintergrund der Erfindung

Die Bier-, Wein- und Bäckerhefe *Saccharomyces cerevisiae* wird aufgrund ihrer Eigenschaft, Zucker zu Ethanol und Kohlendioxid zu fermentieren, schon seit Jahrhunderten für die Brot-, Wein- und Bierherstellung genutzt. In der Biotechnologie findet *S. cerevisiae* neben der Produktion von heterologen Proteinen vor allem in der Ethanolproduktion für industrielle Zwecke Verwendung. Ethanol wird in zahlreichen Industriezweigen als Ausgangssubstrat für Synthesen benutzt. Durch die immer knapper werdenden Ölvorkommen, die steigenden Ölpreise und den stetig steigenden weltweiten Benzinbedarf gewinnt Ethanol als Kraftstoffalternative mehr und mehr an Bedeutung.

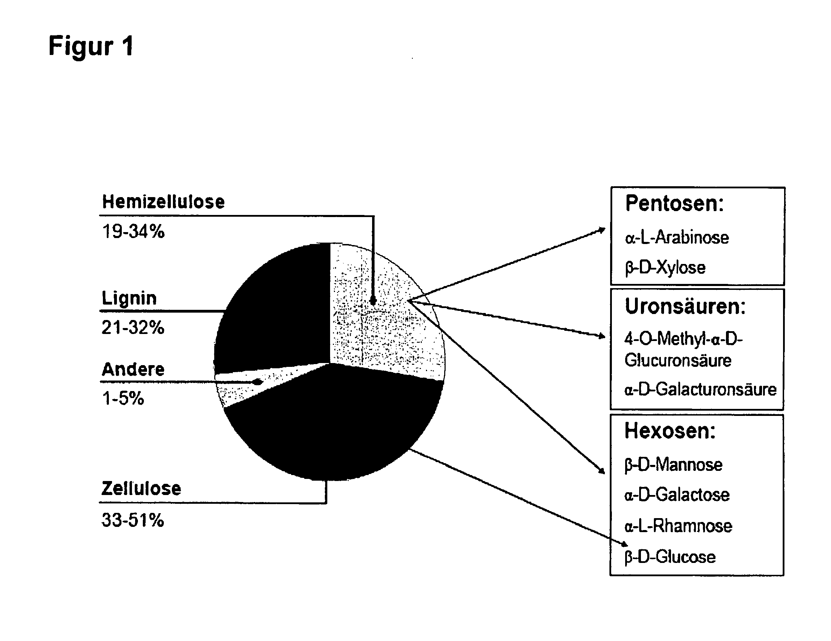

Um eine kostengünstige und effiziente Bioethanol-Produktion zu ermöglichen bietet sich die Verwendung von lignozellulosehaltiger Biomasse, wie z.B. Stroh, Abfälle aus der Holz- und Landwirtschaft und der organische Anteil aus dem alltäglichen Hausmüll, als Ausgangssubstrat an. Diese ist zum einen sehr günstig und zum anderen in großer Menge vorhanden. Die drei größten Bestandteile der Lignozellulose sind Lignin, Zellulose und Hemizellulose. Hemizellulose, nach Zellulose das am zweithäufigsten vorkommende Polymer, ist ein stark verzweigtes Heteropolymer. Es besteht aus Pentosen (L-Arabinose, D-Xylose), Uronsäuren (4-O-Methyl-D-Glucuronsäure, D-Galacturonsäure) und Hexosen (D-Mannose, D-Galactose, L-Rhamnose, D-Glucose) (siehe Figur 1). Hemizellulose lässt sich zwar leichter als Zellulose hydrolysieren, besitzt aber die Pentosen L-Arabinose und D-Xylose, die von der Hefe *S. cerevisiae* normalerweise nicht umgesetzt werden können.

Um Pentosen für Fermentationen nutzen zu können, müssen diese zuerst über die Plasmamembran in die Zelle hinein gelangen. Obwohl *S. cerevisiae* nicht in der Lage ist, D-Xylose zu metabolisieren, kann es diese in die Zelle aufnehmen. *S. cerevisiae* besitzt aber keine spezifischen Transporter. Der Transport findet mit Hilfe der zahlreichen Hexosetransporter statt. Die Affinität der Transporter zu D-Xylose ist aber deutlich niedriger als die zu D-Glucose (Kotter und Ciriacy, 1993). In Hefen, die D-Xylose metabolisieren können, wie z.B. *P. stipitis, C. shehatae* oder *P. tannophilus* (Du Preez et al., 1986), gibt es sowohl unspezifische niederaffine Transporter, die D-Glucose transportieren, als auch spezifische hochaffine Protonen-Symporter nur für D-Xylose (Hahn-Hagerdal *et al.*, 2001).

In früheren Versuchen konnten einige Hefen, wie zum Beispiel *Candida tropicalis, Pachysolen tannophilus, Pichia stipitis, Candida shehatae* gefunden werden, die von Natur aus L-Arabinose fermentieren oder zumindest assimilieren können. Diesen fehlt jedoch die Fähigkeit, L-Arabinose zu Ethanol zu fermentieren ganz oder sie besitzen nur eine sehr geringe Ethanolausbeute (Dien et al., 1996).

### Verwertung von L-Arabinose

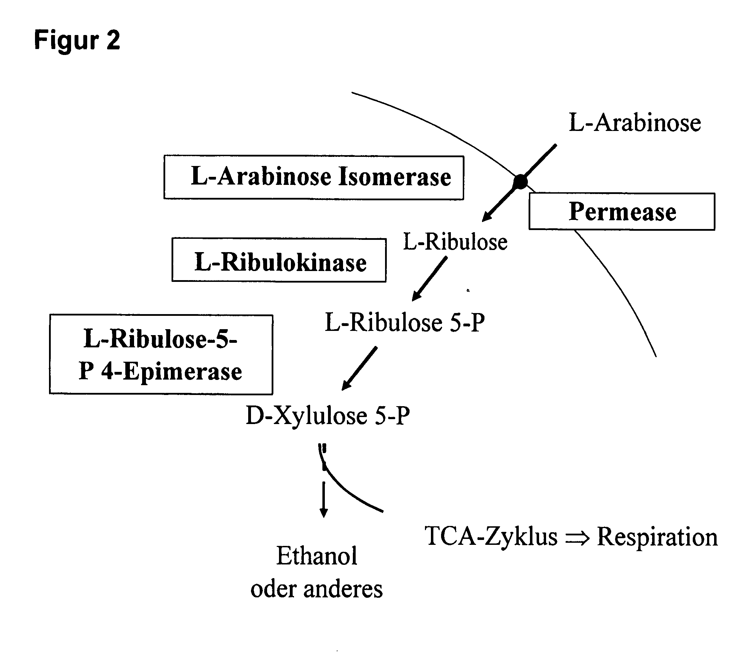

Damit die Pentose L-Arabinose von *S. cerevisiae* metabolisiert werden kann, muss sie über Transportproteine in die Zelle aufgenommen und über drei enzymatische Schritte zu dem Metaboliten D-Xylulose-5-Phosphat umgesetzt werden. Diese drei enzymatischen Schritte können der Hefe durch heterolog exprimierte Gene zur Verfügung gestellt werden. D-Xylulose-5-Phosphat stellt ein Intermediat des Pentosephosphatweges dar und kann in der Zelle unter anaeroben Bedingungen weiter zu Ethanol abgebaut werden kann (siehe Figur 2).

Becker und Boles (2003) beschreiben das Engineering und die Selektion eines Laborstammnes von *S. cerevisiae,* der in der Lage ist, L-Arabinose zum Wachstum zu verwenden und zu Ethanol zu fermentieren. Dies war möglich durch die Überexpression eines bakteriellen L-Arabinose-Stoffwechselweges, bestehend aus *Bacillus subtilis* AraA and *Escherichia coli* AraB and AraD and simultaner Überrexpression der L-Arabinose-transportierenden Hefe-Galaktose-Permease in dem Hefestamm. Molekulare Analyse des selektierten Stammes zeigte, dass die entscheidende Voraussetzung für eine Verwendung von L-Arabinose eine niedrigere Aktivität von L-Ribulokinase ist. Von diesem Hefestamm wird jedoch unter anderem ein sehr langsames Wachstum berichtet. (siehe Figur 2)

WO 2006/096130 A1 (Boles et al.) beschreibt Arabinose- und Xylose-fermentierende *S. cervisiae* Stämme, die Gene des Xylose- und Arabinose-Stoffwechselweges enthalten, wobei der Arabinose-Stoffwechselweg aus den Genen für *B. subtilis* L-Arabinose-Isomerase (araA), *E. coli* L-Ribulokinase (araB) und *E. coli* L-Ribulose-5-P-4-Epimerase (araD) besteht.

WO 98/50524 A1 offenbart die Entwicklung von Plasmiden, welche die *E. coli* Gene des Xylose- und Arabinose-Stoffwechselweges enthalten, und zwar Xylose-Isomerase, Xylulokinase, L-Arabinose-Isomerase, L-Ribulokinase und L-Ribulose-5-P-4-Epimerase, Transaldolase und Transketolase. Die Plasmide werden zur Transformation von *Zymomonas-*Stämmen verwendet, um diese transformierten Stämme in die Lage zu versetzen, auf Xylose und Arabinose zu wachsen und in Ethanol umzuwandeln.

Bisher war es nur möglich, die nativen Gene bakterieller Arabinose-Stoffwechselwege, die für die Arabinose-Umsetzung in *S. cerevisiae* essentiell sind, auf einzelnen Plasmiden zu exprimieren bzw. einzeln in das Genom der Hefe zu integrieren (Karhumaa et al, 2006). Dass heisst, jede Hefetransformande mit einem funktionellen Arabinose-Stoffwechselweg enthielt mindestens drei Plasmide oder die Gene im rDNA-Lokus integriert (Becker und Boles, 2003; Karhumaa et al, 2006).

Das Vorhandensein der Gene auf unterschiedlichen Plasmiden bringt mehrere Nachteile mit sich. Zum einen stellen gleichzeitig vorhandene Plasmide für die Hefezellen eine zusätzliche Belastung dar ("Plasmid-Last", Review zu *E. coli* von Bailey (1993)). Zum anderen weisen die verwendeten Plasmide starke Homologien in ihren Sequenzen auf, so dass es über homologe Rekombination zu Informationsverlusten innerhalb der Plasmide kommen kann (Wiedemann, 2005). Die Hauptnachteile beim Einsatz von Plasmiden liegen jedoch darin, dass sie ohne Selektionsdruck nicht stabil in den Stämmen vorhanden bleiben und dass sie nicht für den industriellen Einsatz geeignet sind.

Zudem wäre es für industrielle Anwendungen ideal, wenn der verwendete Mikroorganismus alle im Medium vorhanden Zucker umsetzten könnte. Da die zur Zeit industriell eingesetzte Hefen nicht in der Lage sind, die im Medium vorhandene Arabinose umzusetzen, wäre es von großem Vorteil, den Stämmen diese zusätzliche Fähigkeit stabil zu verleihen.

WO 03/008593 A2 zeigt die Aminosäuresequenz einer L-Arabinose-Isomerase (araA) aus *Clostridium acetobutylicum* (SEQ ID NO. 9).
Noelling et al. (2001) beschreiben die Genomsequenz und vergleichende Analysen des Lösungsmittel-produzierenden Bakteriums *Clostridium acetobutylicum,* aus dessen Genom araA kloniert wurde.

In der NCBI-Datenbank wird unter der Zugangsnummer AAU41894 die Aminosäuresequenz einer L-Arabinose-Isomerase (araA) aus *Bacillus licheniformis* ATCC 14580 gezeigt. Veith et al. (2004) beschreiben die komplette Nukleinsäuresequenz von *Bacillus licheniformis* DSM13, aus dessen Genom araA kloniert wurde.

Aufgabe der vorliegenden Erfindung ist es daher, Mittel zur Verfügung zu stellen, die die aus dem Stand der Technik bekannten Nachteile der einzelnen Einbringung der Gene eines bakteriellen L-Arabinose-Stoffwechselweges in Wirtszellen überwinden und insbesondere für Industriehefestämme verwendet werden können.

Diese Aufgabe wird durch die Erfindung, wie in den Patentansprüchen beansprucht, gelöst.

Die Aufgabe wird erfindungsgemäß gelöst durch zur Verfügung stellen von Wirtszellen enthaltend Nukleinsäuremoleküle, umfassend drei Nukleinsäuresequenzen, die jeweils für ein Polypeptid eines bakteriellen L-Arabinose-Stoffwechselweges kodieren.

Eine erfindungsgemäße Wirtszelle ist eine Pilzzelle.

Eine erfindungsgemäße Wirtszelle ist bevorzugt eine Hefezelle, wie *Saccharomyces* species, *Kluyveromyces* sp., *Hansenula* sp., *Pichia* sp. oder *Yarrowia* sp..

Bei einem Nukleinsäuremolekül, das in einer erfindungsgemäßen Wirtszelle enthalten ist, handelt es sich um ein rekombinantes Nukleinsäuremolekül. Des Weiteren umfassen die Nukleinsäuremoleküle dsDNA, ssDNA, PNA, CNA, RNA oder mRNA oder Kombinationen davon.

Der "L-Arabinose-Stoffwechselweg" oder "bakterielle L-Arabinose-Stoffwechselweg", wie er z.B. in *E. coli* vorkommt, wird in Figur 2 dargestellt. Dieser Stoffwechselweg beinhaltet die 3 Enzyme: L-Arabinose-Isomerase, L-Ribulokinase und L-Ribulose-5-P-4-Epimerase. Die Gene, die für diese Enzyme kodieren, werden araA, araB und araD genannt. Die L-Arabinose-Isomerase setzt L-Arabinose zu L-Ribulose um, welches durch die L-Ribulokinase weiter zu L-Ribulose-5-Phosphat metabolisiert wird. Die L-Ribulose-5-P-4-Epimerase wandelt schließlich L-Ribulose-5-Phosphat zu D-Xylulose-5-Phosphat um. Durch die in der Hefezelle heterolog exprimierten Gene des L-Arabinose-Stoffwechselweges, insbesondere des bakteriellen L-Arabinose-Stoffwechselweges, entsteht das Zwischenmetabolit D-Xylulose-5-Phosphat. D-Xylulose-5-Phosphat stellt ein Intermediat des Pentosephosphatweges dar und kann in einer Hefezelle unter anaeroben Bedingungen weiter zu Ethanol abgebaut werden. Enzyme des Xylose-Stoffwechselweges werden auch in Pilzen gefunden, diese und andere aus Eukaryonten isolierte Enzyme können als Enzyme für den L-Arabinose-Stoffwechselweg ebenfalls verwendet werden.

Bei den drei Nukleinsäuresequenzen der in einer erfindungsgemäßen Wirtszelle enthaltenen Nukleinsäuremoleküle, die jeweils für ein Polypeptid eines bakteriellen L-Arabinose-Stoffwechselweges kodieren, handelt es sich um araA (L-Arabinose-Isomerase), araB (L-Ribulokinase) und araD (L-Ribulose-5-P-4-Epimerase), wobei die Nukleinsäuresequenz für araA aus *Bacillus licheniformis* oder *Clostridium acetobutylicum* abstammt.

Die in einer erfindungsgemäßen Wirtszelle enthaltenen Nukleinsäuremoleküle umfassen Nukleinsäuresequenzen, die mit der natürlich vorkommenden Nukleinsäuresequenz identisch sind oder für eine Verwendung in einer Wirtszelle Codon-optimiert sind.

Jede Aminosäure wird auf Genebene durch ein Codon verschlüsselt. Jedoch gibt es mehrere verschiedene Codons, die für eine einzelne Aminosäure codieren. Der genetische Code ist folglich degeneriert. Die bevorzugte Codonwahl für eine entsprechende Aminosäure ist von Organismus zu Organismus verschieden. So kann es bei heterolog exprimierten Genen zu Problemen kommen, wenn der Wirtsorganismus bzw. die Wirtszelle einen sehr unterschiedlichen Codon-Gebrauch aufweist. Das Gen kann überhaupt nicht oder nur langsam exprimiert werden. Sogar in Genen von verschiedenen Stoffwechselwegen innerhalb eines Organismus ist ein unterschiedlicher Codon-Gebrauch festzustellen. Von den Glykolyse-Genen aus *S. cerevisiae* ist bekannt, dass sie stark exprimiert werden. Sie weisen einen stark restriktiven Codon-Gebrauch auf. Die Anpassung des Codon-Gebrauchs der bakteriellen Gene des Arabinose-Stoffwechselweges an den Codon-Gebrauch der Glykolyse-Gene aus *S. cerevisiae* führt zu einer Verbesserung des Arabinose-Umsatzes in Hefe.

Bei der Codon-Optimierung haben sich die Erfindern nicht nach den gängigen Plattformen der synthetischen Gene Designer zur heterologen Expression gerichtet (wie beispielsweise Synthetic Gene Designer, wie beschrieben in Wu *et al.* 2006), sondern die Codon-Optimierung spezifisch an den Codon-Gebrauch der Glykolyse-Gene der Hefe angepasst. Die Glykolyse-Gene der Hefe haben einen sehr restriktiven Codon-Gebrauch, der auf die Häufigkeit der entsprechenden tRNA ausgerichtet ist. Die Glykolyse-Gene benutzen hauptsächlich Codons für die es hohe Konzentrationen der entsprechenden tRNAs gibt, was sich dann in einer erhöhten Translationseffizienz und Genexpression niederschlägt (Bennetzen und Hall, 1982, Hoekema *et al.*, 1987). Die gängigen synthetischen Gene Designer orientieren sich dahingegen mehr an dem durchschnittlichen Codongebrauch aller Gene eines Organismus, nicht nur der hochexprimierten, und berücksichtigen außerdem noch andere Faktoren, wie beispielsweise Stabilität. Entsprechend führt eine Codon-Optimierung mit Hilfe einer solchen elektronischen Plattform, wie beispielsweise in Wu *et al.* 2006 genannt, zu einer völlig anderen Nukleinsäuresequenz als der in dieser Patentanmeldung offenbarten.

Erfindungsgemäß sind mindestens zwei der drei in den erfindungsgemäßen Wirtszellen enthaltenen Nukleinsäuresequenzen, besonders bevorzugt alle drei Nukleinsäuresequenzen für eine Verwendung in einer Wirtszelle Codon-optimiert.

Die Nukleinsäuresequenz für araB (L-Ribulokinase) und die Nukleinsäuresequenz für araD (L-Ribulose-5-P-4-Epimerase) stammt bevorzugt aus *E. coli* ab. Dabei umfasst die Nukleinsäuresequenz für araB bevorzugt eine Nukleinsäuresequenz mit der SEQ ID NO: 1 und die Nukleinsäuresequenz für araD bevorzugt eine Nukleinsäuresequenz mit der SEQ ID NO: 2.

Bei der Nukleinsäuresequenz mit der SEQ ID NO: 1 handelt es sich um die Gensequenz des offenen Leserahmens (ORF) von *araB^{mut}* aus *E. coli* in einer Codon-optimierten Form.

Bei der Nukleinsäuresequenz mit der SEQ ID NO: 2 handelt es sich um die die Gensequenz des offenen Leserahmens (ORF) von *araD* aus *E. coli* in einer Codon-optimierten Form.

Die Nukleinsäuresequenz für araA (L-Arabinose-Isomerase) stammt aus *Bacillus licheniformis* oder *Clostridium acetobutylicum* ab.

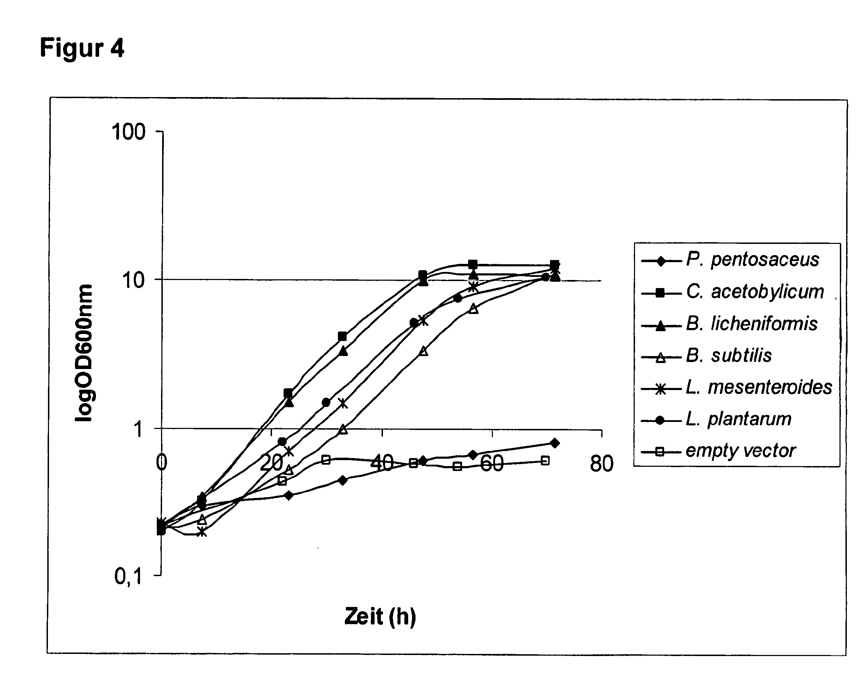

Diese L-Arabinose-Isomerasen sind vorteilhaft für das Wachstum von Hefetransformanden auf Arabinose-Medium. In Beispiel 1 wird gezeigt (siehe auch Figur 4), dass verglichen mit der Isomerase aus *B. subtilis,* besonders die Expression der L-Arabinose-Isomerase von *C. acetobutylicum* und von *B. licheniformis* signifikant das Wachstum von Hefetransformanden auf Arabinose-Medium verbesserten.

Dabei umfasst die Nukleinsäuresequenz für araA bevorzugt eine Nukleinsäuresequenz mit der SEQ ID NO: 3, 4 oder 5.

Bei der Nukleinsäuresequenz mit der SEQ ID NO: 3 handelt es sich um die Gensequenz des offenen Leserahmens (ORF) von *araA* aus *Bacillus licheniformis* in einer Codon-optimierten Form.

Bei der Nukleinsäuresequenz mit der SEQ ID NO: 4 handelt es sich um die Gensequenz des offenen Leserahmens (ORF) von *araA* aus *Bacillus licheniformis*.

Bei der Nukleinsäuresequenz mit der SEQ ID NO: 5 handelt es sich um die Gensequenz des offenen Leserahmens (ORF) von *araA* aus *Clostridium acetobutylicum.*

Bei den Nukleinsäuresequenzen mit der SEQ ID NO: 4 und 5 handelt es sich demzufolge um natürlich vorkommende Nukleinsäuresequenzen.

In einer besonders bevorzugten Ausführungsform umfasst ein in einer erfindungsgemäßen Wirtszelle enthaltenes Nukleinsäuremolekül die Nukleinsäuresequenz mit der SEQ ID NO: 1, die Nukleinsäuresequenz mit der SEQ ID NO: 2 sowie die Nukleinsäuresequenz mit der SEQ ID NO: 3, 4 oder 5. Am meisten bevorzugt wird eine erfindungsgemäße Wirtszelle enthaltend ein Nukleinsäuremolekül, das die Nukleinsäuresequenz mit der SEQ ID NO: 1, die Nukleinsäuresequenz mit der SEQ ID NO: 2 sowie die Nukleinsäuresequenz mit der SEQ ID NO: 3 umfasst.

Hefetransformanden mit den zwei Codon-optimierten Genen der Kinase (araB, SEQ ID NO: 1) und Epimerase (araD, SEQ ID NO: 2) sowie Hefetransformanden, bei denen alle drei Gene Codon-optimiert sind (araB: SEQ ID NO: 1, araD: SEQ ID NO: 2 und araA: SEQ ID NO: 3), zeigen in Arabinose-haltigen Medium einen deutlichen Wachstumsvorteil in Arabinose-haltigen Medium gegenüber Hefetransformanden mit nur einem Codon-optimierten Gen. Die Stämme zeigen eine deutlich verkürzte Lag-Phase und wachsen wesentlich schneller zu ihrer maximalen optischen Dichter heran. (siehe Beispiel 2). Die Kombination von drei Codon-optimierten Genen ermöglicht rekombinanten *S. cerevisiae* Zellen eine wesentlich effizientere L-Arabinose Verwertung.

In einer besonders bevorzugten Ausführungsform ist das Nukleinsäuremolekül in das Genom einer erfindungsgemäßen Wirtszelle stabil integriert.

In einer Ausführungsform ist das in der erfindungsgemäßen Wirtszelle enthaltene Nukleinsäuremolekül in einer Expressionskassette oder einem Expressionsvektor umfasst.

Die Expressionskassetten umfassen weiterhin bevorzugt Promotor- und Terminatorsequenzen.

Promotorsequenzen können ausgewählt sein aus HXT7, verkürzter HXT7, PFK1, FBA1, PGK1, ADH1 und TDH3.

Terminatorsequenzen können ausgewählt sein aus CYC1, FBA1, PGK1, PFK1, ADH1 und TDH3.

Dabei wird es bevorzugt, dass jeweils unterschiedliche Paare aus Promotor- und Terminatorsequenzen die drei Nukleinsäureseuqnzen kontrollieren. Dies ist erforderlich, um eine mögliche homologe Rekombination zwischen den Promotor- bzw. den Terminatorregionen/-sequenzen zu vermeiden.

Die Paare aus Promotor- und Terminatorsequenzen können ausgewählt sein aus HXT7- oder verkürzter HXT7-Promotor und CYC1-Terminator, PFK1-Promotor und FBA1-Terminator sowie FBA1-Promotor und PGK1-Terminator.

Eine Nukleinsäuresequenz für araA kann von dem HXT7- oder verkürzter HXT7-Promotor und dem CYC1-Terminator kontrolliert werden.

Eine Nukleinsäuresequenz für araB kann von dem PFK1-Promotor und dem FBA1-Terminator kontrolliert werden.

Eine Nukleinsäuresequenz für araD kann von dem FBA1-Promotor und dem PGK1-Terminator kontrolliert werden.

Für nähere Ausführungen siehe dazu auch Beispiel 3.

Die Expressionskassetten können weiterhin 5' und/oder 3' Erkennungssequenzen umfassen, wie beispielsweise-Erkennungssequenzen der Enzyme *PacI* und *AscI*.

Die Expressionsvektoren können weiterhin einen Selektionsmarker umfassen.

Ein Selektionsmarker kann ausgewählt sein aus einem Leucin-Marker, einem Uracil-Marker oder einem dominanten Antibiotika-Marker. Ein dominanter Antibiotika-Marker kann ausgewählt sein aus Geneticin, Hygromycin und Nourseothricin.

Beispiele für Expressionsvektoren sind p425H7synthAra, pRS303X, p3RS305X oder p3RS306X.

Für nähere Ausführungen siehe dazu auch Beispiel 3.

Für industrielle Anwendungen wäre es ideal, wenn der verwendete Mikroorganismus alle im Medium vorhanden Zucker umsetzten könnte. Da die zur Zeit eingesetzte Hefen nicht in der Lage sind, die im Medium vorhandene Arabinose umzusetzen, wäre es von großem Vorteil, den Stämmen diese zusätzliche Fähigkeit stabil zu verleihen. Um dies zu erreichen, ist ein Expressionsvektor mit Genen eines Arabinose-Stoffwechselweges von großem Nutzen. Dieser kann dann stabil genomisch integriert werden und kann die Umsetzung von Arabinose in Industriestämmen gewährleisten.

Mit dieser Erfindung ist es gelungen (siehe auch Beispiele), einen Vektor zu konstruieren, der eine Expressionskassette mit drei Genen für einen Arabinose-Stoffwechselweg, insbesondere einen bakteriellen Arabinose-Stoffwechselweg codiert. Hiermit kann die Problematik umgangen werden, die entstehen kann, wenn mehrere Plasmide gleichzeitig in einer Zelle vorhanden sind ("Plasmid-Last", Review zu *E. coli* von Bailey (1993)). Zum anderen wird eine stabile genomische Integration der Arabinose-Stoffwechselweg-Gene ermöglicht. Arbeiten von Becker (2003) und Wiedemann (2005) zeigten bereits die Problematik in der Konstruktion einer Expressionskassette der Arabinose-Stoffwechselweg-Gene sowie deren einzelne stabile genomische Integration.

Durch die Auswahl der Promotoren und Terminatoren, in Kombination mit der Verwerdung der verbesserten L-Arabinose Isomerase und den Codon-optimierten Versionen der beteiligten Gene, konnte es geschafft werden, Wirtszellen enthaltend diese funktionelle Expressionskassette zu konstruieren.

Die von den Erfindern konstruierte und in einer erfindungsgemäßen Wirtszelle enthaltene Expressionskassette mit den drei Genen stellt einen hervorragenden Ausgangspunkt für eine direkte genomische Integration dar sowie ermöglicht eine Subklonierung in die integrativen Plasmide der Serie pRS303X, pRS305X und pRS306X (Taxis und Knop, 2006).

Des Weiteren waren eine Vielzahl von experimentellen Hürden und Schwierigkeiten beim Klonieren der drei Gene mit den unterschiedlichen Promotoren und Terminatoren zu bewältigen, die sich im größeren Detail auch aus den Beispielen und Figuren ergeben.
- Auffinden einer L-Arabinose Isomerase, die in Hefe besser, wie etwa effizienter, funktioniert.
- Die Klonierung der Isomerase gestaltete sich sehr schwierig und zeitaufwendig.
- Bei dem in den erfindungsgemäßen Wirtszellen enthaltenen Vektor handelt sich um den ersten beschriebenen Vektor, der alle essentiellen Gene für die Arabinose-Verwertung in Hefe enthält.
- Der Vektor enthält alle Gene in funktioneller Form und ermöglicht der rekombinanten Hefe ein sehr gutes Arabinosewachstum. Funktionalität sowie sehr gutes Arabinosewachstum waren nicht unbedingt zu erwarten.

Für industrielle Anwendungen wäre es ideal, wenn der verwendete Mikroorganismus alle im Medium vorhanden Zucker umsetzten könnte. Da die zurzeit eingesetzte Hefen nicht in der Lage sind, die im Medium vorhandene Arabinose umzusetzen, wäre es von großem Vorteil, den Stämmen diese zusätzliche Fähigkeit stabil zu verleihen. Um dies zu erreichen, kann erfindungsgemäß ein hierin definiertes Nukleinsäuremolekül, eine hierin definierte Expressionskassette oder ein hierin definierter Expressionsvektor stabil genomisch integriert werden und kann die Umsetzung von Arabinose in Industriestämmen gewährleisten. Die Verwendung der erfindungsgemäß verwendeten Nukleinsäuremoleküle gewährleistet eine sehr effiziente Arabinose-Umsetzung in den Industriestämmen. Das einzelne Einführen der Gene des bakteriellen L-Arabinose-Stoffwechselweges brachte bislang die Schwierigkeit mit sich, dass die Gene nicht in ihrem optimalen Verhältnis zu einander vorlagen. Die Transformationen waren zeitaufwendig und erzielten oft nicht die gewünschte Effizienz der Arabinose-Umsetzung. Zusätzlich waren die verliehenen Eigenschaften oft nicht stabil. Hingegen ermöglicht die erfindungsgemäß verwendete Expressionskassette bzw. der erfindungsgemäß verwendete Expressionsvektor die schnelle und funktionelle Einbringung des bakteriellen L-Arabinose-Stoffwechselweges. Durch die Auswahl der Promotoren konnten die Gene gemeinsam auf einem Nukleinsäuremolekül, einer Expressionskassette bzw. einem Expressionsvektor vereinigt werden. Die erfindungsgemäße Integration des hierin definierten Nukleinsäuremoleküls, der hierin definierten Expressionskassette bzw. des hierin definierten Expressionsvektors gewährleistet weiterhin eine effiziente Arabinose-Umsetzung.

Eine erfindungsgemäße Wirtszelle ist eine Pilzzelle und bevorzugt eine Hefezelle, wie *Saccharomyces* species, *Kluyveromyces* sp., *Hansenula* sp., *Pichia* sp. oder *Yarrowia* sp..

Eine erfindungsgemäße Wirtszelle kann ausgewählt sein aus BWY1, CEN.PK113-7D, Red Star Ethanol Red und Fermiol.

Die Aufgabe wird weiterhin erfindungsgemäß gelöst durch zur Verfügung stellen von Verfahren zur Produktion von Bioethanol. Ein erfindungsgemäßes Verfahren umfasst die Expression eines Nukleinsäuremoleküls, wie hierin definiert, einer Expressionskassette, wie hierin definiert oder eines Expressionsvektors, wie hierin definiert in einer Wirtszelle.

Dabei wird das Verfahren in einer erfindungsgemäßen Wirtszelle durchgeführt.

Die Aufgabe wird weiterhin erfindungsgemäß gelöst durch die Verwendung einer erfindungsgemäßen Wirtszelle zur Produktion von Bioethanol.

Die Aufgabe wird weiterhin erfindungsgemäß gelöst durch die Verwendung einer erfindungsgemäßen Wirtszelle zur rekombinanten Fermentation von Pentose-haltigem Biomaterial.

Für die Verfahren und Verwendungen wird auch auf die Beispiele und Figuren verwiesen. Aus den in Beispiel 2 gezeigten Fermenationsergebnissen geht hervor, dass insbesondere die Codon-optimierten Gene von araA, araB und araD den Hefetranformanden eine effizientere Arabinose-Umsetzung ermöglichen. Das Resultat daraus ist eine schnellere Umsetzung des Zuckers sowie eine deutlich höhere Ethanol-Ausbeute.

Die Anmeldung beschreibt weiterhin ein Polypeptids, ausgewählt aus der Gruppe von
a. einem Polypeptid, das zu mindestens 70%, beispielsweise mindestens 80% identisch zu der Aminosäuresequenz ist, die von SEQ ID NO: 3, 4 oder 5 kodiert wird, und eine *in vitro* und/oder *in vivo* Pentose-Isomerasefunktion aufweist,
b. einer natürlich auftretenden Variante eines Polypeptids umfassend die Aminosäuresequenz, die von SEQ ID NO: 3, 4 oder 5 kodiert wird, die eine *in vitro* und/oder *in vivo* Pentose-Isomerasefunktion aufweist,
c. einem Polypeptid, das identisch zu der Aminosäuresequenz ist, die von SEQ ID NO: 3, 4 oder 5 kodiert wird, und eine *in vitro* und/oder *in vivo* Pentose-Isomerasefunktion aufweist, und
d. einem Fragment des Polypeptids aus a., b. oder c., umfassend ein Fragment von mindestens 100, 200 oder 300 kontinuierlichen Aminosäuren der Aminosäuresequenz, die von SEQ ID NO: 3, 4 oder 5 kodiert wird.

Ein solches Polypeptid kann auch ausgewählt sein aus der Gruppe von
a. einem Polypeptid, das zu mindestens 70%, beispielsweise mindestens 80% identisch zu der Aminosäuresequenz nach SEQ ID NO: 6 oder 7 ist und eine *in vitro* und/oder *in vivo* Pentose-Isomerasefunktion aufweist,
b. einer natürlich auftretenden Variante eines Polypeptids umfassend die Aminosäuresequenz nach SEQ ID NO: 6 oder 7, die eine *in vitro* und/oder *in vivo* Pentose-Isomerasefunktion aufweist,
c. einem Polypeptid, das identisch zu der Aminosäuresequenz nach SEQ ID NO: 6 oder 7 ist und eine *in vitro* und/oder *in vivo* Pentose-Isomerasefunktion aufweist, und
d. einem Fragment des Polypeptids aus a., b. oder c., umfassend ein Fragment von mindestens 100, 200 oder 300 kontinuierlichen Aminosäuren nach SEQ ID NO: 6 oder 7.

Ein solches Polypeptid kann ein Polypeptid umfassen, das zu mindestens 90%, beispielsweise 95% identisch zu der Aminosäuresequenz ist, die von SEQ ID NO: 3, 4 oder 5 kodiert wird, und eine *in vitro* und/oder *in vivo* Pentose-Isomerasefunktion aufweist.

Ein solches Polypeptid kann ein Polypeptid umfassen, das zu mindestens 90%, beispielsweise 95% zu der Aminosäuresequenz nach SEQ ID NO: 6 oder 7 ist und eine *in vitro* und/oder *in vivo* Pentose-Isomerasefunktion aufweist.

Bei der Aminosäuresequenz mit der SEQ ID NO. 6 handelt es sich um die Aminosäuresequenz der *Bacillus licheniformis* L-Arabinose-Isomerase (araA). Diese Aminosäuresequenz wird von den Nukleinsäuresequenzen mit den SEQ ID NOs. 3 oder 4 kodiert.

Bei der Aminosäuresequenz mit der SEQ ID NO. 7 handelt es sich um die Aminosäuresequenz der *Clostridium acetobutylicum* L-Arabinose-Isomerase (araA). Diese Aminosäuresequenz wird von der Nukleinsäuresequenz mit der SEQ ID NO. 5 kodiert.

Die Pentose ist Arabinose, insbesondere L-Arabinose.

Das Polypeptid stammt aus einem Bakterium, insbesondere aus *Bacillus licheniformis* oder *Clostridium acetobutylicum* ab.

Diese L-Arabinose-Isomerasen sind vorteilhaft für das Wachstum von Hefetransformanden auf Arabinose-Medium. Verschiedene Experimente wiesen darauf hin, dass die bisher verwendete L-Arabinose-Isomerase aus *B. subtilis* einen limitierenden Schritt in dem Arabinose-Abbau in Hefe darstellt (Becker und Boles, 2003; Wiedemann, 2003; Karhumaa et al, 2006; Sedlak und Ho, 2001). In Beispiel 1 wird gezeigt (siehe auch Figur 4), dass verglichen mit der Isomerase aus *B. subtilis,* besonders die Expression der L-Arabinose-Isomerase von C. *acetobutylicum* und von B. *licheniformis* signifikant das Wachstum von Hefetransformanden auf Arabinose-Medium verbessern.

Die Anmeldung beschreibt weiterhin ein isoliertes Nukleinsäuremolekül, das für ein Polypeptid kodiert.

Die Anmeldung beschreibt weiterhin eine Wirtszelle, die ein solches isoliertes Nukleinsäuremolekül enthält.

Das Polypeptid, isolierte Nukleinsäuremolekül und die Wirtszelle eignen sich zur Produktion von Bioethanol und zur rekombinanten Fermentation von Pentose-haltigem Biomaterial.

Die Anmeldung beschreibt weiterhin Wirtszellen, die eine oder mehrere zusätzliche Modifikationen, wie Nukleinsäuremoleküle enthalten.

Bei einer solchen zusätzlichen Modifikation handelt es sich um eine Wirtszelle, die ein TAL1 (Transaldolase)-Gen überexprimiert, wie beispielsweise von den Erfindern in der EP 1 499 708 B1 beschrieben.

Bei einer weiteren solchen zusätzlichen Modifikation handelt es sich zum anderen um eine Wirtszelle, die eine Nukleinsäure kodierend für ein spezifisches L-Arabinose-Transportergen (araT) enthält, wie insbesondere ein spezifisches L-Arabinose-Transportergen aus dem Genom von *P. stipitis*, wie von den Erfindern in der deutschen Patentanmeldung DE 10 1006 060 381.8, eingereicht am 20. Dezember 2006, beschrieben.

Weitere Biomasse mit signifikanten Mengen an Arabinose (Quelle der Daten: U.S. Department of Energy http://www.eere.energy.gov/biomass/progs/search1.cgi):

| Art der Biomasse | L-Arabinose [%] |
|---|---|
| Switchgras | 3,66 |
| Grosses Bartgras | 3,55 |
| Hohes Schwingelgras | 3,19 |
| Robinie | 3 |
| Corn Stover | 2,69 |
| Weizen Stroh | 2,35 |
| Zuckerrohr Bagasse | 2,06 |
| Chinesischer Buschklee | 1,75 |
| Futterhirse | 1,65 |

Auch für deren Verwertung sind die erfindungsgemäßen Nukleinsäuren, Expressionskassetten, Expressionsvektoren und Wirtszellen von großer Bedeutung.

Nutzungsmöglichkeiten der erfindungsgemäßen Nukleinsäuren, Expressionskassetten, Expressionsvektoren und Wirtszellen sind zum einen die Produktion von Bioethanol und die Herstellung von hochwertigen Vorläufer-Produkten für weitere chemische Synthesen.

Folgende Auflistung stammt aus der Studie "Top Value Added Chemicals From Biomass" (siehe wwwl.eere.energy.gov/biomass/pdfs/35523.pdf). Hierbei wurden 30 Chemikalien als besonders wertvoll eingestuft, welche aus Biomasse hergestellt werden können.

| Anzahl der C-Atome | Top 30 Kandidaten |
|---|---|
| 1 | Wasserstoff, Kohlenmonoxid |
| 2 | |
| 3 | Glycerol, 3-Hydroxypropionsäure, Milchsäure, Malonsäure, Propionsäure, Serin |
| 4 | Acetoin, Asparaginsäure, Fumarsäure, 3-Hydroxybutyrolacton, Apfelsäure, Bernsteinsäure, Threonin |
| 5 | Arabitol, Furfural, Glutaminsäure, Itaconsäure, Levulinsäure, Prolin, Xylitol, Xylonsäure |
| 6 | Aconitsäure, Citrat, 2,5-Furandicarboxylsäure, Glucaricsäure, Lysin, Levoglucosan, Sorbitol |

Sobald diese Chemikalien aus Lignozellulose durch Biokonversion (z.B. Fermentationen mit Hefen) hergestellt werden, ist es wichtig, die erfindungsgemäßen Nukleinsäuren, Expressionskassetten, Expressionsvektoren und Wirtszellen zur Verfügung zu haben.

Die vorliegende Erfindung wird in den folgenden Figuren, Sequenzen und Beispielen weiter verdeutlicht, ohne jedoch darauf beschränkt zu sein. Die zitierten Referenzen sind hiermit durch Bezugnahme vollständig aufgenommen. In den Sequenzen und Figuren wird gezeigt:
SEQ ID NO: 1 zeigt die Gensequenz des offenen Leserahmens (ORF) von *araB^{mut}* aus *E. coli* in einer Codon-optimierten Form.
SEQ ID NO: 2 zeigt die Gensequenz des offenen Leserahmens (ORF) von *araD* aus *E. coli* in einer Codon-optimierten Form.
SEQ ID NO: 3 zeigt die Gensequenz des offenen Leserahmens (ORF) von *araA* aus *B. licheniformis* in einer Codon-optimierten Form.
SEQ ID NO: 4 zeigt die Gensequenz des offenen Leserahmens (ORF) von *araA* aus *B. licheniformis.*
SEQ ID NO: 5 zeigt die Gensequenz des offenen Leserahmens (ORF) von *araA* aus *C. acetobutylicum*.
SEQ ID NO. 6 zeigt die Aminosäuresequenz der *Bacillus licheniformis* L-Arabinose-Isomerase (araA). Diese Aminosäuresequenz wird bevorzugt von den Nukleinsäuresequenzen mit den SEQ ID NOs. 3 oder 4 kodiert.
SEQ ID NO. 7 zeigt die Aminosäuresequenz der *Clostridium acetobutylicum* L-Arabinose-Isomerase (araA). Diese Aminosäuresequenz wird bevorzugt von der Nukleinsäuresequenz mit der SEQ ID NO. 5 kodiert.

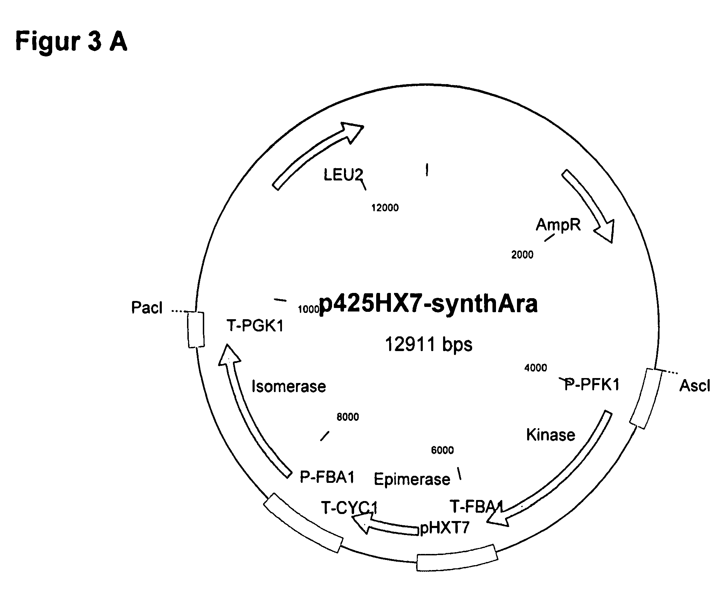

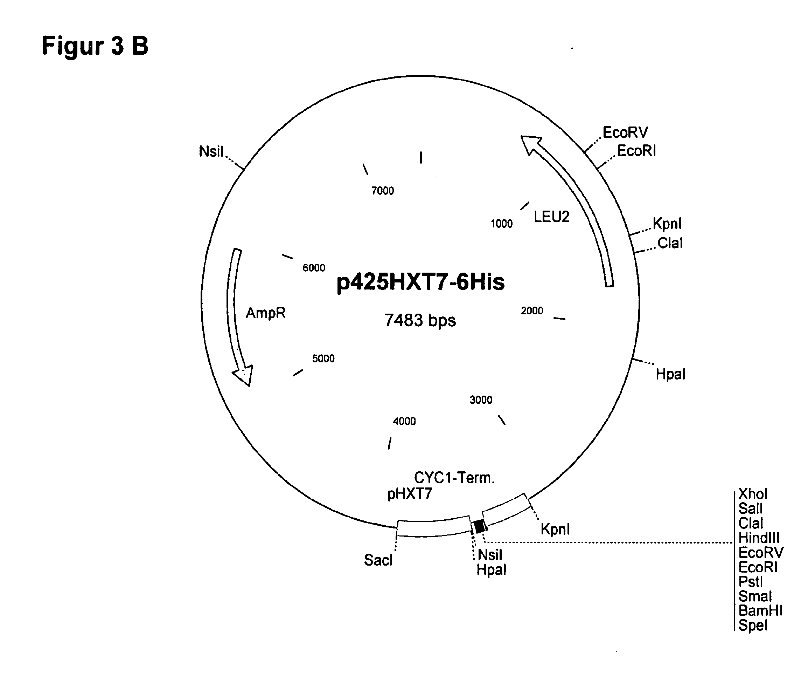

**Figur 1****.** *Zusammensetzung der Biomasse*.
   Biomasse besteht aus Zellulose, Hemizellulose und Lignin. Die am zweithäufigsten vorkommende Hemizellulose ist ein aus Pentosen, Uronsäuren und Hexosen bestehendes stark verzweigtes Polymer. Die Hemizellulose besteht zu einem großen Anteil aus den Pentosen Xylose und Arabinose.
**Figur 2****.** *Schema für die Umsetzung von L-Arabinose in rekombinantner S. cerevisiae durch Integration eines bakteriellen L-Arabinose-Stoffwechselweges.*
**Figur 3** **:** *Verwendete Vektoren und deren Aufbau*.
   Ausgangsplasmid für die Konstruktion des Vektors p425H7synthAra (**Figur 3** **A**) war das Plasmid p425HXT7-6HIS (**Figur 3** **B**). Die offenen Leserahmen der Codon-optimierten Gene von *araA* aus *B. licheniformis* und *araB^{mut}* und *araD* aus *E. coli* wurden amplifiziert und hinter verschiedene Promotoren und Terminatoren in das Plasmid p425HXT7-6HIS kloniert. Die Primer wurden so gewählt, daß die entstandene Expressionskassette von den Schnittstellen der Enzyme *Pac*I und *Asc*I flankiert wird. Hierbei entstand das Plasmid p425H7synthAra, welches einen Leucin-Marker besitzt.
**Figur 4** *Wachstum auf Arabinose unter Verwendung der verschiedenen L-Arabinose-Isomerase-Gene*.
   Wachstumskurven von rekombinanten *S. cerevisiae* Stämmen, die den bakteriellen L-Arabinose-Stoffwechsel mit verschiedenen L-Arabinose-Isomerasen enthalten. Wachstumstests wurden in 5 ml SM-Medium mit 2 % Arabinose unter aeroben Bedingungen durchgeführt. Getestet wurde die L-Arabinose-Isomerasen von *C. acetobutylicum, B. licheniformis, P. pentosaceus, L. plantarum* und *L. mesenteroides*. Als Vergleich diente die L-Arabinose-Isomerase aus *B. subtilis* und der leere Vektor p423HXT7-6HIS.

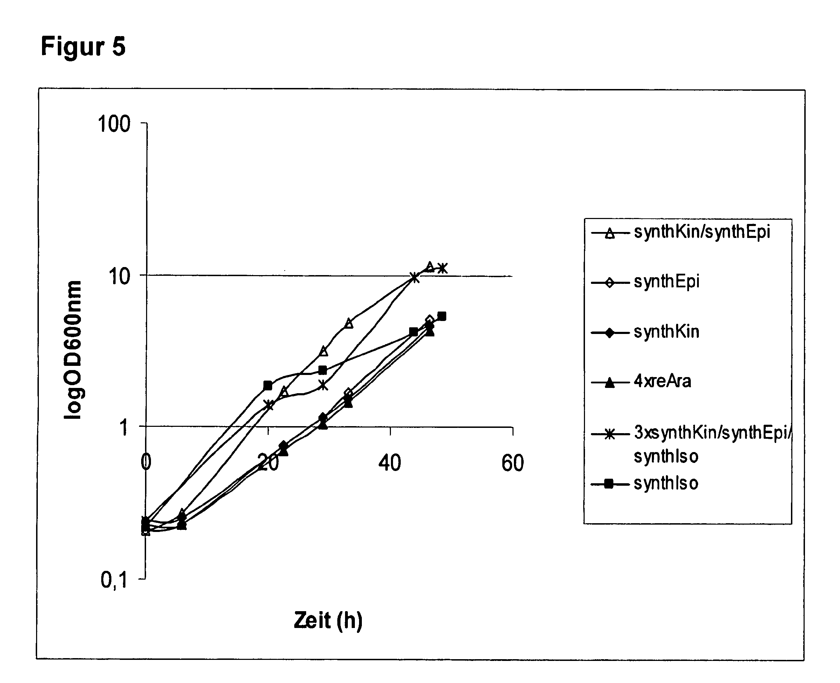

**Figur 5** *Wachstum auf Arabinose unter Verwendung der Codon-optimierten Arabinose-Stoffwechselweg-Gene*.
   Wachstumskurven von rekombinanten *S. cerevisiae* Stämmen, die den bakteriellen L-Arabinose-Stoffwechsel mit unterschiedlichen Kombinationen aus Codon-optimierten Genen sowie den Genen mit Original-Sequenzen enthalten. Wachstumstests wurden in 5 ml SM-Medium mit 2 % Arabinose unter aeroben Bedingungen durchgeführt. Getestet wurden die Kombinationen, indem jeweils eines der Codon-optimierten Gene enthalten war, sowie die Kombination, indem alle drei Codon-optimierten Gene vorhanden waren. Zusätzlich wurde die Kombination getestet, in der das Codon-optimierte Gen der Kinase und der Epimerase vorhanden waren. Als Vergleich diente ein rekombinanter Hefestamm mit den vier Genen mit den Original-Sequenzen.

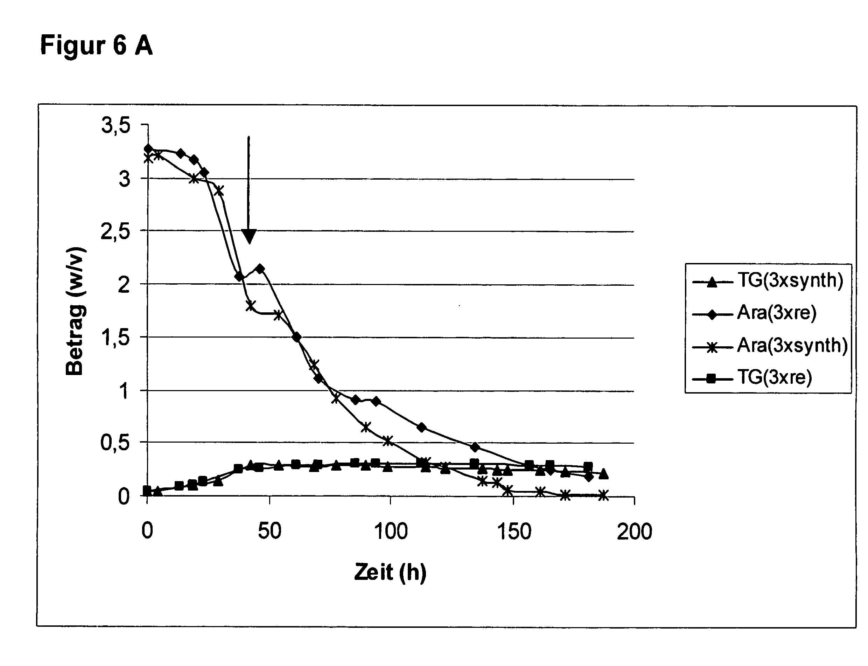

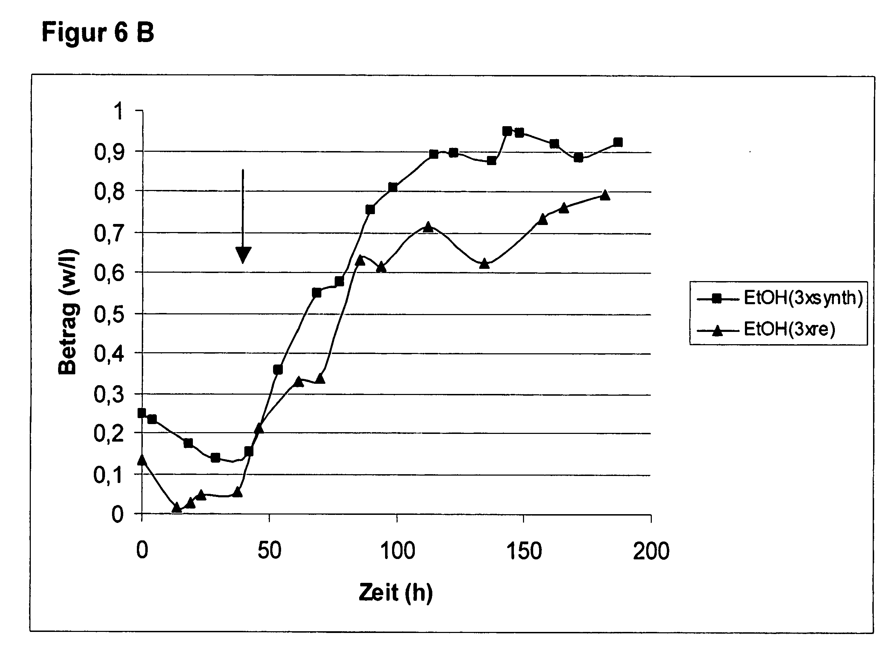

**Figur 6** *Ethanolbildung unter Verwendung der Codon-optimierten Arabinose-Stoffwechselweg-Gene*.
   Gezeigt werden die Ergebnisse von HPLC-Analysen der Medienüberstände zweier Fermentationen. Die eine Fermentation wurde mit Stamm BWY1 durchgeführt, der die Plasmide p423H7synthIso, p424H7synthKin, p425H7synthEpi und pHL125^{re} (3xsynth) besitzt. In der anderen Fermentation wurde Stamm BWY1 getestet, welcher die Plasmide p423H7araABs^{re}, p424H7araB^{re}, p425H7araD^{re} und pHL125^{re} (3xre) enthält. Die Fermentationen wurden in SFM-Medium mit 3 % L-Arabinose durchgeführt. Die Stämme wurden bis zum Erreichen einer hohen optischen Dichte aerob im Fermenter angezogen. Anschließend wurde die Fermentation auf anaerobe Bedingungen umgestellt (nach 48 Stunden). In den Kurven sind Arabinose-Verbrauch und Ethanol-Produktion dargestellt.

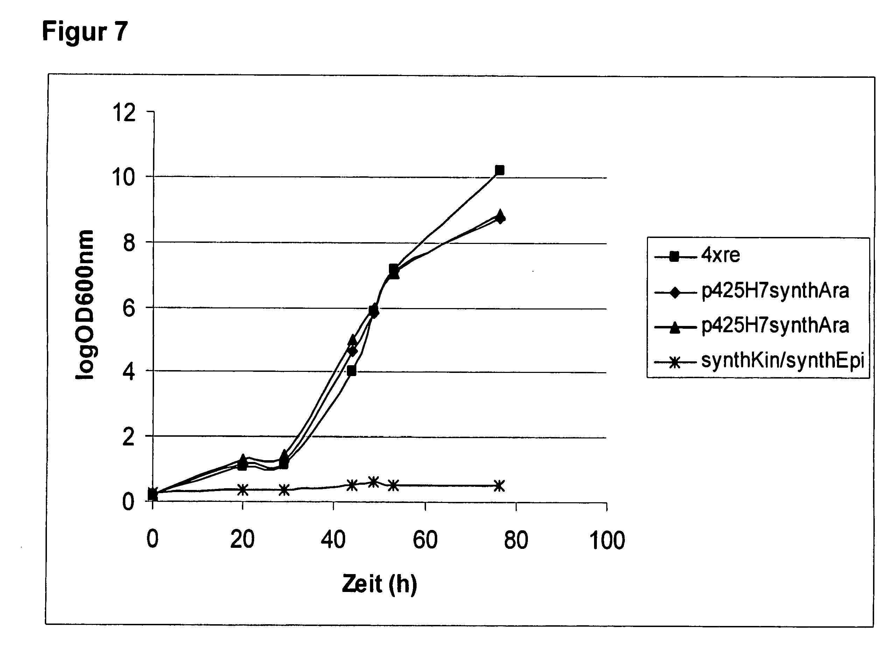

**Figur 7** *Wachstum auf Arabinose unter Verwendung des konstruieren Expressionsplasmids p425H7-synthAra*.
   Wachstumskurven von rekombinanten *S. cerevisiae* Stämmen, die den bakteriellen L-Arabinose-Stoffwechsel in Form des Vektors p425H7-synthAra enthalten. Wachstumstests wurden in 5 ml SC-Medium mit 2 % Arabinose unter aeroben Bedingungen durchgeführt. Als Vergleich diente ein rekombinanter Hefestamm mit den Plasmiden p423H7araABs^{re}, p424H7araB^{re}, p425H7araD^{re} und pHL125^{re}, der in 5 ml SM-Medium mit 2 % Arabinose getestet wurde.

### Beispiele

### Methoden

### 1. Stämme und Medien

### -Bakterien

- *E. coli* SURE (Stratagene)
- *E. coli* DH5α (Stratagene)
- *Bacillus licheniformis* (DSMZ)
- *Clostridium acetobutylicum* (DSMZ)
- *Leuconostoc mesenteroides* (DSMZ)
- *Pediococcus pentosaceus* (DSMZ)
- *Lactobacillus plantarum* (DSMZ)

Vollmedium LB 1% Trypton, 0,5 % Hefeextrakt, 0,5% NaCl, pH 7,5 (siehe Maniatis, 1982). Für die Selektion auf eine plasmidkodierte Antibiotikaresistenz wurde dem Medium nach dem Autoklavieren 40µg/ml Ampicillin zugesetzt. Feste Nährmedien enthielten zusätzlich 2% Agar. Die Anzucht erfolgte bei 37°C.

### -Hefe

### Stamm BWY1:

BWY1 basiert auf dem Stamm JBY25 (*MATa leu2-3,112 ura3-52 trp1-289 his3-Δ1MAL2-8c* SUC2+ unbekannte Mutationen für besseres Wachstum auf Arabinose); der Stamm JBY25 wurde weiter selektioniert und besitzt weitere Mutationen für verbessertes Wachstum auf L-Arabinose unter Sauerstoff-limitierten Bedingungen (Wiedemann, 2005)
- synthetisches Komplettselektivmedium SC:
   0,67% Yeast nitrogen base w/o amino acids, pH6,3, Aminosäure/Nukleobase-Lösung, Kohlenstoffquelle in der jeweils angegeben Konzentration
- synthetisches Minimalselektivmedium SM:
   0,16% Yeast nitrogen base w/o amino acid and ammonium sulphate, 0,5% Ammoniumsulfat ,20mM Kalium-dihydrogenphosphat, pH6,3, Kohlenstoffquelle in der jeweils angegeben Konzentration
- synthetisches Fermentationsmedium (Mineralmedium) SFM:
   (Verduyn *et al.*, 1992), pH 5,5
   Salze: (NH₄)₂SO₄, 5g/l; KH₂P0₄, 3g/l; MgSO₄*7H₂O, 0,5g/l
   Spurenelemente: EDTA, 15mg/l, ZnSO₄*4,5mg/l; MnCl₂*4H₂O, 0,1mg/l;
   CoCl₂*6H₂O, 0,3 mg/l; CuSO₄, 0,192 mg/ml; Na₂MoO₄*2H₂O, 0,4 mg/l;
   CaCl₂*2H₂O, 4,5 mg/l; FeSO₄*7H₂O, 3 mg/l; H₃BO₃, 1 mg/l; KI, 0,1 mg/l
   Vitamine: Biotin, 0,05 mg/l; p-Aminobenzoesäure, 0,2 mg/l; Nicotinsäure, 1 mg/l;
   Calciumpantothenat, 1 mg/l; Pyridoxin-HCL, 1 mg/l; Thiamin-HCL, 1 mg/l; Minositol, 25 mg/l

Konzentration der Aminosäuren und Nukleobasen im synthetischen Komplettmedium (nach Zimmermann, 1975): Adenin (0,08mM), Arginin (0,22mM), Histidin (0,25mM), Isoleucin (0,44mM), Leucin (0,44mM), Lysin (0,35mM), Methionin (0,26mM), Phenylalanin (0,29mM), Tryptophan (0,19mM), Threonin (0,48mM), Tyrosin (0,34mM), Uracil (0,44mM), Valin (0,49mM). Als Kohlenstoffquelle wurden L-Arabinose und D-Glucose eingesetzt. Feste Voll- und Selektivmedien enthielten zusätzlich 1,8 % Agar. Die Anzucht der Hefezellen erfolgte bei 30°C. Das für die Fermentationen eingesetzte synthetische Mineralmedium enthielt Salze, Spurenmetalle und Vitamine in den oben aufgelisteten Konzentrationen und L-Arabinose als Kohlenstoffquelle. Von den Spurenmetallen und den Vitaminen wurde eine Stammlösung angesetzt. Beide Lösungen wurden sterilfiltriert. Beide wurden bei 4°C gelagert. Für die Herstellung der Spurenmetalllösung war der pH-Wert von entscheidender Rolle. Die verschiedenen Spurenelemente mußten in der obigen Reihenfolge nacheinander in Wasser vollständig gelöst werden. Nach jeder Zugabe mußte der pH-Wert mit KOH auf 6,0 eingestellt werden bevor das nächste Spurenelement zugegeben werden konnte. Am Ende wurde der pH-Wert mit HCL auf 4,0 justiert. Um Schaumbildung zu vermeide, wurde dem Medium 200µl Antischaum (Antifoam2004, Sigma) zugegeben. Da die Versuche unter anaeroben Bedingungen durchgeführt wurden, mußte dem Medium nach dem Autoklaviern noch 2,5 ml/l einer Tween80-Ergosterol-Lösung zugegeben werden. Diese besteht aus 16,8 g Tween80 und 0,4 g Ergosterol, welche mit Ethanol auf 50 ml aufgefüllt und darin gelöst wurden. Die Lösung wurde sterilfiltriert. Die Salze und der Antischaum wurden gemeinsam mit dem kompletten Fermenter autoklaviert. Die Arabinose wurde getrennt vom restlichen Medium autoklaviert. Nach Abkühlen des Mediums wurden die Spurenelemente sowie die Vitamine hinzugegeben.

### 2. Plasmide

| Plasmid | Quelle/Referenz | Beschreibung |
|---|---|---|
| p423HXT7-6HIS (=p423H7) | Becker und Boles, 2003 | 2µ-Expressionsplasmid für die Überexpression verschiedener Gene und für die Fusionierung der *E. coli* L-Arabinose Isomerase mit einem His₆-Epitop; *HIS3-*Selektionsmarkergen, verkürzter HXT7-Promotor und *CYC1*-Terminator (Hamacher *et al.*, 2002) |
| p424HXT7-6HIS (=p424H7) | Becker und Boles, 2003 | 2µ-Expressionsplasmid für die Überexpression verschiedener Gene und für die Fusionierung der mutierten und der Wildtyp *E. coli* L-Ribulokinase mit einem His₆-Epitop; *TRP1*-Selektionsmarkergen, verkürzter *HXT7-*Promotor und *CYC1*-Terminator (Hamacher *et al.*, 2002) |
| p425HXT7-6HIS (=p425H7) | Becker und Boles, 2003 | 2µ-Expressionsplasmid für die Überexpression verschiedener Gene; LEU2-Selektionsmarkergen, verkürzter *HXT7*-Promotor und *CYC1*-Terminator (Hamacher *et al.*, 2002) |
| p426HXT7-6HIS (=p426H7) | Hamacher *et al.*, 2002 | 2µ-Expressionsplasmid zur Überexpression von Genen und zur Herstellung eines His₆-Epitop; *USA3-*Selektionsmarkergen, verkürzter HXT7-Promotor und *CYC1*-Terminator |
| p423H7araABs^{re} | Becker und Boles, 2003 | *B.subtilis araA* in p423HXT7-His, re-isoliert aus JBY25-4M |
| p424H7araB | Becker und Boles, 2003 | *E. coli araB* in p423HXT7-His |
| p424H7araB^{re} | Becker und Boles, 2003 | *E. coli araB* in p423HXT7-His; re-isoliert aus JBY25-4M, Mutation in *araB,* welche Arabinose-Wachstum ermöglicht |
| p425H7araD^{re} | Becker und Boles, 2003 | *E. coli araD* in p425HXT7-His; re-isoliert aus JBY25-4M |
| | | |
| p423H7-synthIso | | *B. licheniformis araA* Codon-optimiert in p423HXT7-His |
| p424H7-synthKin | | *E. coli araB* Codon-optimiert in p424HXT7-His, mit Mutation in *araB* |
| p425H7-synthEpi | | *E. coli araD* Codon-optimiert in p425HXT7-His |
| p425H7-synthAra | | 2µ-Plasmid mit den Codon-optimierten Genen *araA, araB^{mut}* und *araD; araA* unter Kontrolle des *FBA1-*Promotor und dem *PGK1* Terminator, *araB^{mut}* unter der Kontrolle des *PFK1* Promotor und dem *FBA1* Terminator, und *araD* unter Kontrolle des verkürzten *HXT7* Promotor und dem *CYC1* Terminator, *LEU2*-Selektionsmarkergen |
| pHL125^{re} | Liang und Gaber, 1996 | 2µ-Plasmid mit dem *GAL2*-Gen exprimiert hinter dem *ADH1*-Promotor, *URA3*-Selektionsmarkergen; re-isoliert aus JBY25-4M |

### 3. Transformation:

### - Transformation von E. coli

Die Transformation der *E. coli* Zellen erfolgte durch die Elektroporationsmethode nach Dower *et al.* (1988) und Wirth (1993) mittels eines Easyject prima Geräts (EQUIBO).

### - Transformation von S. cerevisiae

Die Transformation von *S. cerevisiae* Stämmen mit Plasmid-DNA bzw. DNA-Fragmenten erfolgte nach der Lithiumacetat-Methode von Gietz und Woods (1994).

### 4. Präparation von DNA

### - Isolierung von Plasmid-DNA aus E. coli

Die Isolierung von Plasmid-DNA aus *E. coli* erfolgte nach der Methode der alkalischen Lyse von Birnboim und Doly (1979), modifiziert nach Maniatis *et al.* (1982) oder alternativ mit dem "QIAprep Spin Miniprep Kit" der Firma Qiagen.

Hochreine Plasmid-DNA für Sequenzierungen wurde mit dem "Plasmid Mini Kit" der Firma Qiagen nach Angaben des Herstellers präpariert.

### - Isolierung von Plasmid-DNA aus S. cerevisiae

Die Zellen einer stationären Hefekultur (5ml) wurden durch Zentrifugation geerntet, gewaschen und in 400µl Puffer P1 (Plasmid Mini Kit, Firma Qiagen) resuspendiert. Nach Zugabe von 400µl Puffer P2 und ²/₃ Volumen Glasperlen (Ø 0,45mm) erfolgte der Zellaufschluss durch 5-minütiges Schütteln auf einem Vibrax (Vibrax-VXR von Janke & Kunkel oder IKA). Der Überstand wurde mit ½ Volumen Puffer P3 versetzt, gemischt und für 10min auf Eis inkubiert. Nach einer 10-minütigen Zentrifugation bei 13000rpm wurde durch Zugabe von 0,75ml Isopropanol zum Überstand die Plasmid-DNA bei Raumtemperatur gefällt. Die durch Zentrifugation für 30min bei 13000rpm pelletierte DNA wurde mit 70%igem Ethanol gewaschen, getrocknet und in 20µl Wasser resuspendiert. 1µl der DNA wurde für die Transformation in *E. coli* eingesetzt.

### - Bestimmung der DNA-Konzentration

Die DNA-Konzentration wurde spektralphotometrisch in einem Wellenlängenbereich von 240-300nm gemessen. Liegt die Reinheit der DNA, bestimmt durch den Quotient E₂₆₀ₙₘ/E₂₈₀ₙₘ, bei 1,8, so entspricht die Extinktion E₂₆₀ₙₘ=1,0 einer DNA-Konzentration von 50µg dsDNA/ml (Maniatis *et al.*, 1982).

### - DNA-Amplifikation mittels PCR

### Verwendung des Phusion^{™} High Fidelity Systems

Die Polymerasekettenreaktion erfolgte in einem Gesamtvolumen von 50µl mit dem "Phusion^{™} High Fidelity PCR System" der Firma Finnzymes nach Angaben des Herstellers. Jeder Ansatz bestand aus 1-10ng DNA oder 1-2 Hefekolonien als Synthesevorlage, 0,2mM dNTP-Mix, 1xPuffer 2 (enthält 1,5mM MgCl₂), 1U Polymerase und je 100pmol der entsprechenden Oligonukleotidprimer. Die PCR-Reaktion wurde in einem Thermocycler der Firma Techne durchgeführt und die PCR-Bedingungen nach Bedarf wie folgt gewählt:

| | | |
|---|---|---|
| 1. 1x | 30sec, 98°C | Denaturierung der DNA |
| 2. 30x | 10sec, 98°C | Denaturierung der DNA |
| | 30sec, 56-62°C | AnnealingBindung der Oligonukleotide an die DNA |
| | 0,5-1min, 72°C | DNA-Synthese/Elongation |
| 3. 1x | 7min, 72°C | DNA-Synthese/Elongation |

Nach dem ersten Denaturierungsschritt wurde die Polymerase zugegeben ("hot start PCR"). Die Anzahl der Syntheseschritte, die Annealingtemperatur und die Elongationszeit wurden an die spezifischen Schmelztemperaturen der verwendeten Oligonukleotide bzw. an die Größe des zu erwarteten Produkts angepaßt. Die PCR-Produkte wurden durch eine Agarosegelelektrophorese überprüft und anschließend aufgereinigt.

### - DNA-Aufreinigung von PCR-Produkten

Die Aufreinigung der PCR-Produkte erfolgte mit dem "QIAquick PCR Purification Kit" der Firma Qiagen nach Herstellerangaben.

### - Gelelektrophoretische Auftrennung von DNA-Fragmenten

Die Auftrennung von DNA-Fragmenten mit einer Größe von 0,15-20kb erfolgte in 0,5-1%igen Agarosegelen mit 0,5µg/ml Ethidiumbromid. Als Gel- und Laufpuffer wurde 1xTAE-Puffer (40mM Tris, 40mM Essigsäure, 2mM EDTA) verwendet (Maniatis *et al.*, 1982). Als Größenstandard diente eine mit den Restriktionsendonukleasen *Eco*RI und *Hin*dIII geschnittene Lambda-Phagen-DNA. Die DNA-Proben wurden vor dem Auftragen mit ¹/₁₀ Volumen Blaumarker (1xTAE-Puffer, 10% Glycerin, 0,004% Bromphenolblau) versetzt und nach der Auftrennung durch Bestrahlung mit UV-Licht (254nm) sichtbar gemacht.

### - Isolierung von DNA-Fragmenten aus Agarosegelen

Das gewünschte DNA-Fragment wurde aus dem TAE-Agarosegel unter langwelligem UV-Licht (366nm) ausgeschnitten und mit dem "QIAquick Gel Extraction Kit" der Firma Qiagen nach Angaben des Herstellers isoliert.

### 5. Enzymatische Modifikation von DNA

### DNA-Restriktion

Sequenzspezifische Spaltung der DNA mit Restriktionsendonukleasen wurden unter den vom Hersteller empfohlenen Inkubationsbedingungen für 1 Stunde mit 2-5U Enzym pro µg DNA durchgeführt.

### Beispiel 1: Screen nach besseren L-Arabinose-Isomerasen

### A) Durchführung des Screens

Verschiedene Experimente wiesen darauf hin, dass die L-Arabinose-Isomerase aus *B. subtilis* einen limitierenden Schritt in dem Arabinose-Abbau in Hefe darstellt (Becker und Boles, 2003; Wiedemann, 2003; Karhumaa et al, 2006; Sedlak und Ho, 2001). Um eine Verbesserung des Arabinose-Stoffwechselweges zu erzielen, wurden fünf L-Arabinose Isomerasen aus verschiedenen Organismen getestet.

Hierzu wurde genomische DNA aus den Organismen C. *acetobutylicum, B. licheniformis, P. pentosaceus, L. plantarum* und *L. mesenteroides* isoliert (siehe dazu "Isolierung von Plasmid-DNA aus *S. cerevisiae*"). Die Zellen wurden herangezogen, geerntet und in Puffer aufgenommen. Der Zellaufschluss erfolgte mit Hilfe von Glasperlen. Anschließend wurde die DNA gefällt, gewaschen und für die PCR eingesetzt. Der offene Leserahmen (ORF) von *araA* von den genannten Organismen wurde mit Primern amplifiziert, die zusätzlich homologe Bereiche zum *HXT7*-Promotor bzw. *CYC1*-Terminator aufwiesen. Zusammen mit dem *EcoR*I/*BamH*I linearisierten Vektor p423HXT7-6His wurden die erhaltenen PCR-Produkte in Hefe transformiert und über *in vivo*-Rekombination in das Plasmid zwischen den *HXT7-*Promotor bzw. *CYC1*-Terminator kloniert. Die Sequenz der erhaltenen Plasmide wurde über Restriktionsanalyse verifiziert. Weiterhin sollte von den neuen Isomerasen die Funktionalität und deren Effekt auf den Arabinose-Umsatz untersucht werden.

Hierzu wurden rekombinante Hefestämme hergestellt, die eine der neuen Isomerasen enthielten sowie die restlichen bakteriellen Arabinose-Stoffwechselweg-Gene (p424H7araB^{re}, p425H7araD^{re} und pHL125^{re}).

### B) Wachstumsverhalten

Das Wachstum der Stämme wurde unter aeroben Bedingungen in Arabinose-haltigem Medium getestet. Als Vergleich diente der rekombinante Hefestamm, welcher die Isomerase aus *B. subtilis* enthielt. Als Negativkontrolle wurde ein Hefestamm konstruiert, der den leeren Vektor p423HXT7-6HIS besaß.

Die Stämme mit den verschiednen Isomerase-Plasmiden wurden in SM-Medium mit 2 % Arabinose herangezogen und mit einer OD₆₀₀ₙₘ= 0,2 in 5 ml SM-Medium mit 2 % Arabinose angeimpft. Die Inkubation erfolgte in Reagenzgläsern auf einem Schüttelkolben unter aeroben Bedingungen bei 30°C. Es wurde mehrfach Proben zur Bestimmung der optischen Dichte genommen.

Die Ergebnisse sind in Figur 4 gezeigt. Es zeigte sich, dass verglichen mit der Isomerase aus *B. subtilis*, besonders die Expression der L-Arabinose-Isomerase von *C. acetobutylicum* und von *B. licheniformis* signifikant das Wachstum der Hefetransformanden auf Arabinose Medium verbesserten.

### Beispiel 2: Codon-Optimierung der Gene für den Arabinose-Abbau in Hefe

### A) Codon-Optimierung von Genen nach dem Codongebrauch der Glycolysegene aus S. cerevisiae

Der bevorzugte Codongebrauch der Glycolysegene aus *S. cerevisiae* wurde ermittelt und ist in Tabelle 1 aufgelistet. Es wurde der ORF der Gene *araA* und *araB^{mut}* aus *E. coli* Codon-optimiert, sowie der ORF des Genes *araA* aus *B. licheniformis.* Das heißt, die Sequenzen der offenen Leserahmen wurden dem unten angeführten bevorzugten Codon-Gebrauch angepaßt. Die Proteinsequenz der Enzyme blieb unverändert. Die Gene wurden bei einer externen Firma synthetisiert und in getrockneter Form in firmeneigenen Hausvektoren geliefert.

Nähere Angaben zu der Synthese von Genen finden sich unter http://www.sloning.com/.

**Tabelle1: Bevorzugter Codon-Gebrauch der Glycolysegene aus S. cerevisiae.**

| Amminosäure bevorzugtes | Codon |
|---|---|
| Ala | GCT |
| Arg | AGA |
| Asn | AAC |
| Asp | GAC, (GAT) |
| Cys | TGT |
| Gln | CAA |
| Glu | GAA |
| Gly | GGT |
| His | CAC |
| Ile | ATT, (ATC) |
| Leu | TTG |
| Lys | AAG |
| Met | ATG |
| Phe | TTC |
| Pro | CCA |
| Ser | TCT, (TCC) |
| Thr | ACC, (ACT) |
| Trp | TGG |
| Tyr | TAC |
| Val | GTT, (GTC) |
| Stopp | TAA |

### B) Einführung der Codon-optimierten Gene in den Stamm BWY1

Um die drei Codon-optimierten Gene in den Stamm BWY1 zu transformieren und zu testen, mussten die Gene in Hefevektoren subkloniert werden. Hierfür wurden der Codon-optimierte *araA*-ORF, der *araB^{mut}*-ORF und der *araD*-ORF mit Primern amplifiziert, bei denen homologe Überhänge zu dem verkürzten *HXT7*-Promotor und dem *CYC1*-Terminator entstanden. Die 2µ Expressionsplasmide p423HXT7-6HIS, p424HXT7-6HIS, p425HXT7-6HIS wurden mit Restriktionsendonukleasen im Bereich zwischen *HXT7*-Promotor und *CYC1*-Terminator linearisiert. Das PCR-Produkt von *araA* wurde mit dem linearisierten p423HXT7-6HIS in Hefe transformiert und über *in vivo*-Rekombination zu dem Plasmid p423H7-synthIso kloniert. Ebenso wurde mit dem PCR-Produkt *araB^{mut}* und dem linearisierten Vektor p424HXT7-6HIS vorgegangen. Es entstand das Plasmid p424H7synthKin. Durch *in vivo*-Rekombination in Hefe von PCR-Produkt *araD* und dem linearisierten Vektor p425HXT7-6HIS entstand Plasmid p425H7-synthIso. Die Plasmide wurden aus Hefe isoliert und in *E. coli* amplifiziert. Nach der Isolierung der Plasmide aus *E. coli* wurden die Plasmide mittels Restriktionsanalyse überprüft. Zum Test auf Funktionalität und zur weiteren Analyse wurde jeweils eines der Plasmide mit den Codon-optimierten Genen zusammen mit den drei Original -re-isolierten Plasmiden in den Hefestamm BWY1 transformiert, so dass alle entstandenen rekombinanten Stämme einen vollständigen Arabinose-Stoffwechselweg enthielten. Zusätzlich wurde die Kombination p424H7synthKin und p42457synthEpi mit den Original- re-isolierten Plasmiden getestet, sowie ein Ansatz, bei der die Hefetransformande alle drei neuen Plasmide besaß. Die Transformation mit den jeweils vier Plasmiden erfolgte gleichzeitig. Die Transformanden wurden auf SM-Medium mit 2 % Glucose ausplattiert. Nach zwei Tagen wurden die erhaltenen Kolonien auf SM-Medium mit 2 %Arabinose ausgestrichen. Als Positivkontrolle diente ein Hefestamm, der die vier Original- re-isolierten Plasmide enthielt.

### C) Wachstumsverhalten

Das Wachstum des Stammes BWY1 mit den unterschiedlichen Plasmidkombinationen aus Codon-optimierten Genen und Original-Genen wurde in Wachstumstests auf Arabinose-haltigem Medium unter aeroben Bedingungen untersucht.

Die Stämme mit den verschiedenen Plasmidkombinationen wurden in SM-Medium mit 2 % L-Arabinose herangezogen und mit einer OD₆₀₀ₙₘ = 0,2 in 5 ml SM-Medium mit 2 % L-Arabinose angeimpft. Die Inkubation erfolgte in Reagenzgläsern unter aeroben Bedingungen bei 30°C. Es wurden mehrfach Proben zur Bestimmung der optischen Dichte entnommen.

Die Ergebnisse der aeroben Wachstumskurve sind in Figur 5 dargestellt. Es ist deutlich zu erkennen, dass rekombinante Hefestämme, die nur eines der optimierten Gene besitzen, gegenüber dem Stamm mit den vier Originalplasmiden kaum oder nur wenig Wachstumsvorteile in Arabinose-haltigen Medium aufweisen. Jedoch zeigten Hefetransformanden mit den zwei optimierten Genen der Kinase und Epimerase sowohl Hefetransformanden mit drei optimierten Genen einen deutlichen Wachstumsvorteil in Arabinose- haltigen Medium. Die Stämme zeigten eine deutlich verkürzte Lag-Phase und wuchsen wesentlich schneller zu ihrer maximalen optischen Dichter heran.

Damit wird gezeigt, dass die Kombination der drei Codon-optimierten Genen den rekombinanten *S. cerevisiae* Zellen, eine wesentlich effizientere L-Arabinose Verwertung ermöglicht.

### D) Ethanol-Produktion

In Figur 6 (A) und (B) werden die Ergebnisse von HPLC-Analysen zweier Fermentationen gezeigt. Ein rekombinanter Hefestamm enthält die Plasmide p423H7synthIso, p424H7synthKin, p425H7synthEpi und pHL125^{re}, der andere Stamm die Plasmide p423H7araABs^{re}, p424H7araB^{re}, p425H7araD^{re} und pHL125^{re}. Die Fermentationen wurden in SFM-Medium mit 3 % L-Arabinose durchgeführt. Figur 6 (A) zeigt den Arabinose-Verbrauch und das Trockengewicht der beiden Stämme. In Figur 6 (B) ist die Ethanol-Produktion beider Stämme dargestellt.

Die Stämme wurden bis zum Erreichen eines Trockengewichts von ca. 2,8 g/l aerob im Fermenter angezogen. Bei Erreichen von genug Zellmasse wurden die Fermentationen auf anaerobe Bedingungen umgestellt. Gezeigt sind die Kurven des Arabinose-Umsatzes sowie die Ethanol-Produktion. Die entstandenen Nebenprodukte Arabitol, Acetat und Glycerin sind nicht aufgeführt, da sie bei beiden Stämmen in vergleichbaren Mengen produziert wurden. Wie aus den Kurven erkennbar ist, setzt bei beiden Stämmen die Ethanol-Produktion direkt nach dem Wechsel auf anaerobe Bedingungen ein (Wechsel zu anaeroben Bedingungen gezeigt in Fig 6 (A) und (B) durch Pfeil). Der schon zu Beginn der Fermentation enthaltene Ethanol im Medium war nicht von den Hefen produziert, sondern stammte aus der Tween80/Ergosterol-Lösung. Unter den am Anfang herrschenden aeroben Bedingungen wurde Ethanol respiratorisch von Hefe abgebaut.

Nach ca. 80 Stunden zeigt der Stamm, der die Arabinose-Stoffwechselweg-Gene in Codonoptimierter Form besitzt, einen deutlich verbesserten Arabinose-Umsatz sowie eine erhöhte Ethanol-Produktion. Die vorhandene Arabinose im Medium ist bereits nach 150 Stunden vollständig verbraucht. Hingegen ist bei dem Stamm mit den Original re-isolierten Plasmiden selbst nach über 180 Stunden noch Arabinose im Medium vorhanden.

Aus den Fermenationsergebnissen geht hervor, dass die Codon-optimierten Gene den Hefetranformanden eine effizientere Arabinose-Umsetzung ermöglichen. Das Resultat daraus ist eine schnellere Umsetzung des Zuckers sowie eine deutlich höhere Ethanol-Ausbeute.

### Beispiel 3: Konstruktion einer Expressionskassette mit drei Genen für den Arabinose-Stoffwechselweg

Der Vektor mit der Expressionskassette mit drei Genen für den Arabinose-Stoffwechselweg wurde konstruiert, um zum einen die Problematik zu umgehen, die entstehen kann, wenn mehrere Plasmide gleichzeitig in einer Zelle vorhanden sind ("Plasmid-Last", Review zu E. coli von Bailey (1993)), und zum anderen, um eine stabile genomische Integration der Arabinose-Stoffwechselweg-Gene zu ermöglichen. Arbeiten von Becker (2003) und Wiedemann (2005) zeigten bereits die Problematik in der Konstruktion einer Expressionskassette der Arabinose-Stoffwechselweg-Gene sowie deren einzelne stabile genomische Integration. Die nun konstruierte Expressionskassette mit den drei Genen stellt einen hervorragenden Ausgangspunkt für eine direkte genomische Integration dar, sowie ermöglicht eine Subklonierung in die integrativen Plasmide der Serie pRS303X, pRS305X und pRS306X (Taxis und Knop, 2006).

### A) Konstruktion der Expressionskassette

Für die Konstruktion der Expressionskassette wurde von dem Plasmid p425H7-synthEpi ausgegangen, in dem die Codon-optimierte Form der Epimerase aus *E. coli* hinter dem verkürzten *HXT7* Promotor und vor dem *CYC1* Terminator exprimiert wurde. Um eine mögliche homologe Rekombination zwischen identischen Promotor- bzw. Terminatorregionen zu vermeiden, sollte der Codon-optimierte *araB^{mut}-* ORF aus *E. coli* zwischen den *PFK1*-Promotor und dem *FBA1*-Terminator exprimiert werden, der Codon-optimierte *araA*-ORF aus *B. licheniformis* zwischen den *FBA1*-Promotor und den *PGK1-*Terminator. Das Plasmid p425H7-synthEpi wurde mit der Restriktionsendonuklease *Sac*I vor dem *HXT7*-Promotor geöffnet, auf ein Agarosegel aufgetragen und aus dem Gel eluiert. Der *araB^{mut}*-ORF wurde per PCR amplifiziert. *PFK1*-Promotor und *FBA1*-Terminator wurden aus genomischer DNA von *S. cerevisiae* amplifiziert, wobei die Primer so gewählt wurden, dass zum einen eine 500bp lange Sequenz des Promotors bzw. eine 300bp lange Sequenz des Terminators synthetisiert wurde und gleichzeitig homologe Überhänge zum Plasmid p425H7-synthEpi bzw. zum *araB^{mut}*-ORF entstanden. Zusätzlich enthielt der Primer, welcher den *PFK1*-Promotor mit den homologen Bereichen zu p425H7-synthEpi amplifizierte eine Sequenz für eine *PacI* Schnittstelle. Zusammen mit dem linearisierten Vektor wurden die drei PCR-Produkte in Hefe transformiert und über *in vivo*-Rekombination in das Plasmid kloniert. Die erfolgreiche Konstruktion des entstandenen Plasmids p425H7synthEpisynthKin wurde über Restriktionsanalyse verifiziert. Die Funktionalität des Vektors wurde getestet. Hierzu wurden Hefestransformanden hergestellt, die die Plasmide p425H7synthEpisynthKin und p423H7araABs^{re} enthielten. Die Transformanden wurden auf Arabinosewachstum getestet. Der Stamm war in der Lage, auf Arabinose-haltigem Medium zu wachsen. Als NegativKontrolle diente ein Hefestamm, der die Vektoren p424H7synthEpi sowie p423HXT7-6HIS enthielt. Dieser war nicht in der Lage, auf dem Medium zu wachsen.

Im nächsten Schritt wurde die Codon-optimierte Form der Isomerase aus *B. licheniformis* in den Vektor integriert. Hierzu wurde Plasmid p425H7synthEpisynthKin mit *NgoM*VI hinter dem *CYC1*-Terminator linearisiert, auf ein Agarosegel aufgetragen und aus dem Gel eluiert. Es wurde eine 500bp lange Sequenz des *FBA1*-Promotors aus genomischer DNA von *S. cerevisiae* amplifiziert, wobei die Primer so gewählt wurden, dass homologe Überhänge zu Plasmid p425H7synthEpisynthKin im *CYC1*-Terminator und zu dem ORF der Codon-optimierten *araA* entstanden. Zusätzlich wurde ein 300bp lange Sequenz des *PGK1-*Terminators aus genomischer DNA von *S. cerevisiae* amplifiziert, bei der ein Primer zu dem ORF der Codon-optimierten *araA* homologe Überhänge besaß und der andere Primer homologe Überhänge zu Plasmid p425H7synthEpisynthKin sowie eine *AscI* Schnittstelle aufwies.

Die erfolgreiche Konstruktion des entstandenen Plasmids p425H7synthAra wurde wieder über Restriktionsanalyse verifiziert und seine Funktionalität überprüft. Der Test auf Funktionalität wurde auf Arabinosewachstum durchgeführt. Hefetransformanden, die das Plasmid p425H7synthAra enthielten, zeigten Wachstum auf Arabinose-haltigem Medium. Wachstumskurven in 5 ml SC-Medium mit 2 % Arabinose wurden durchgeführt. In Figur 7 ist zu erkennen, dass die Transformanden mit Vektor p425H7synthAra vergleichbares Wachstum zeigen wie ein Stamm mit den vier Original reisolierten Plasmiden.

### B) Rolle der Promotoren und Terminatoren

Um eine mögliche homologe Rekombination zwischen den Promotor bzw. den Terminatorregionen zu vermeiden, wurden die drei Gene hinter unterschiedliche Promotoren und Terminatoren kloniert. Besonders wichtig war dabei die Auswahl der Promotoren. Aus vorangehenden Arbeiten (Becker und Boles, 2003) zeigte sich, dass die Gendosis der drei Gene zu einander von entscheidender Bedeutung war. Zusätzlich sollten alle Gene stark exprimiert werden. Aus diesen Gründen fiel die Wahl zum einen auf den verkürzten HXT7-Promotor, der konstitutiv und stark exprimiert wird, sowie die Promotoren PFK1 und FBA1, von denen bekannt ist, dass sie eine starke Expression der Gene bewirken.

### C) Beispiele für Vektoren für die Expressionskassette

Ausgangsplasmid für die Konstruktion von p425H7synthAra war das Plasmid p425H7synthEpi, welches auf dem Plasmid p425HXT7-6HIS basiert. Bei dem Vektor handelt es sich um ein 2µ Expressionsplasmid, welches einen Leucin-Marker besitzt.

Die drei Arabinose-Stoffwechselweg Gene wurden hintereinander unter die Kontrolle von unterschiedlichen Promotoren und Terminatoren in einen Vektor kloniert. Die Expressionskassette ist von den Erkennungssequenzen der Enzyme *PacI* und *AscI* flankiert.

Weitere mögliche Expressionsvektoren sind aus der Serie pRS303X, p3RS305X und p3RS306X. Hierbei handelt es sich um integrative Vectoren, die einen dominanten Antibiotika-Marker besitzen. Nähere Angaben zu diesen Vektoren finden sich bei Taxis und Knop (2006).

### Referenzen

Becker, J. (2003) Konstruktion und Charakterisierung eines L-Arabinose fermentierenden Saccharomyces cerevisiae Hefestammes. Dissertation, Heinrich-Heine-Universität Düsseldorf
Becker, J. und Boles, E. (2003) A modified Saccharomyces cerevisiae strain that consumes L-arabinose and produces ethanol. Appl. Environ. Microbiol. 69:4144-4150
Bailey, J.E. (1993) Host-vector interactions in Escherichia coli. Adv. Biochem Eng. 48.29-52
Bennetzen, J.L. und Hall, B.D. (1982) Codon selection in yeast. J Biol Chem. 257(6):3026-2031.
Birnboim, H.C. und J. Doly (1979) A rapid alkaline extraction procedure for screening recombinant plasmid DNA. Nucl. Acids Res. 7: 1513-1523
Dien, B.S., Kurtzman, C.P., Saha, B.C. and Bothast, R.J. (1996) Screening for L-arabinose fermenting yeasts. Appl. Biochem. Biotechnol. 57-58, 233-42.
Dower, W.J., Miller, J.F. und Ragsdale, C.W. (1988) High efficiency transformation of E. coli by high voltage electroporation. Nucl. Acids Res. 16: 6127-6145
Du Preez, J.C., Bosch, M., and Prior, B.A. (1986) The fermentation of hexose and pentose sugars by Candida shehatae and Pichia stipitis. Appl. Microbiol. Biotechnol. 23: 228-233.
Gietz, R.D. und Woods, R.A. (1994) High efficiency transformation in yeast. In: Molecular Genetics of Yeast: Practical Approaches, J.A. Johnston (Ed.). Oxford University Press pp. 121-134
Hahn-Hägerdal, B., Wahlbom, C.F., Gárdonyi, M., van Zyl, W., Otero, R.R.C. and Jönsson, L.J. (2001) Metabolic engineering of Saccharomyces cerevisiae for xylose utilization. Adv. Biochem. Eng. Biotechnol. 73:53-84.
Hamacher, T., Becker, J., Gárdonyi, M., Hahn-Hägerdal, B. und Boles., E. (2002) Characterization of the xylose-transporting properties of yeast hexose transportes and their influence on xylose utilization. Microbiology 148:2783-2788.
Hoekema A, Kastelein RA, Vasser M, de Boer HA. (1987) Codon replacement in the PGK1 gene of Saccharomyces cerevisiae: experimental approach to study the role of biased codon usage in gene expression. Mol Cell Biol. 7(8):2914-2924.
Karhumaa, K., Wiedemann, B., Hahn-Hägerdal, B., Boles, E. and Gorwa-Grauslund, MF. (2006) Co-utilisation of L-arabinose and D-xylose by laboratory and industrial Saccharomyces cerevisiae strains. Microbial Cell Factories 5(1):18
Kötter, P. and Ciriacy, M. (1993) Xylose fermentation by Sacharomyces cerevisiae. Appl. Microbiol. Biotechnol. 38: 776-783.
Liang, H. and Gaber, R.F. (1996) A novel signal transduction pathway in Saccharomyces cerevisiae defined by Snf3-regulated expression of HXT6. Mol. Biol. Cell 7: 1953-1966.
Maniatis T, Fritsch, E.F und Sambrook, J. (1982) . Molecular cloning. A laboratory manual. Cold Spring Harbor Laboratory, New York.
Noelling J, Breton G, Omelchenko MV, Makarova KS, Zeng Q, Gibson R, Lee HM, Dubois J, Qiu D, Hitti J, Wolf YI, Tatusov RL, Sabathe F, Doucette-Stamm L, Soucaille P, Daly MJ, Bennett GN, Koonin EV, Smith DR. (2001) Genome sequence and comparative analysis of the solvent-producing bacterium Clostridium acetobutylicum. J Bacteriol. 183(16):4823-38.
Sedlak, M. und Ho, N.W.Y. (2001) Expression of E. coli araBAD operon encoding enzymes for metabolizing L-arabinose in Saccharomyces cerevisiae. Enz. Microbiol. 28:16-24
Taxis, C. und Knop, M. (2006) System of centromeric, episomal, and integrative vectors based on drug resistance markers for Saccharomyces cerevisiae. BioTechniques 40, No. 1
Veith B, Herzberg C, Steckel S, Feesche J, Maurer KH, Ehrenreich P, Bäumer S, Henne A, Liesegang H, Merkl R, Ehrenreich A, Gottschalk G. The complete genome sequence of Bacillus licheniformis DSM13, an organism with great industrial potential. J Mol Microbiol Biotechnol. 7(4):204-11.
Verduyn, C., Postma, E., Scheffers, W.A. und Van Dijken, J.P. (1992) Effect of benzoic acid on metabolic fluxes in yeasts: a continuous-culture study on the regulation of respiration and alcoholic fermentation. Yeast 8 (7), 501-17
Wiedemann, B.(2005) Molekulargenetische und physiologische Charakterisierung eines rekombinanten Pentosevergärenden Hefestammes. Diplomarbeit. Johann Wolfgang Goethe-Universität, Frankfurt am Main.
Wirth;R. (1993) Elektroporation: Eine alternative Methode zur Transformation von Bakterien mit Plasmid-DNA. Forum Mikrobiologie 11 (507-515).
Wu G, Bashir-Bello N, Freeland SJ. (2006) The Synthetic Gene Designer: a flexible web platform to explore sequence manipulation for heterologous expression. Protein Expr Purif. 47(2):441-445.
Zimmermann, F. K. (1975) Procedures used in the induction of mitotic recombination and mutation in the yeast Saccharomyces cerevisiae. Mutation Res. 31:71-81

## Patentansprüche

1. Wirtszelle, enthaltend ein Nukleinsäuremolekül, das drei Nukleinsäuresequenzen umfasst, die jeweils für ein Polypeptid eines bakteriellen L-Arabinose-Stoffwechselweges kodieren,
wobei die drei Nukleinsäuresequenzen araA (L-Arabinose-Isomerase), araB (L-Ribulokinase) und araD (L-Ribulose-5-P-4-Epimerase) sind,
wobei die Nukleinsäuresequenz für araA aus *Bacillus licheniformis* oder *Clostridium acetobutylicum* abstammt,
und wobei die Wirtszelle eine Pilzzelle ist.

2. Wirtszelle nach Anspruch 1, die eine Hefezelle, wie *Saccharomyces* species, *Kluyveromyces* sp., *Hansenula* sp., *Pichia* sp. oder *Yarrowia* sp., ist.

3. Wirtszelle nach Anspruch 1 oder 2, wobei die Nukleinsäuresequenz für araB und die Nukleinsäuresequenz für araD aus *E. coli* abstammt.

4. Wirtszelle nach einem der Ansprüche 1 bis 3, wobei die Nukleinsäuresequenz für araA eine Nukleinsäuresequenz mit der SEQ ID NO: 3, 4 oder 5 umfasst.

5. Wirtszelle nach einem der vorangehenden Ansprüche, wobei mindestens zwei der drei Nukleinsäuresequenzen für eine Verwendung in einer Wirtszelle Codon-optimiert sind.

6. Wirtszelle nach einem der vorangehenden Ansprüche, wobei die Nukleinsäuresequenz für araB eine Nukleinsäuresequenz mit der SEQ ID NO: 1 umfasst,
und/oder wobei die Nukleinsäuresequenz für araD eine Nukleinsäuresequenz mit der SEQ ID NO: 2 umfasst.

7. Wirtszelle nach einem der vorangehenden Ansprüche, wobei das Nukleinsäuemolekül die Nukleinsäuresequenz mit der SEQ ID NO: 1, die Nukleinsäuresequenz mit der SEQ ID NO: 2 sowie die Nukleinsäuresequenz mit der SEQ ID NO: 3, 4 oder 5 umfasst.

8. Wirtszelle nach einem der vorangehenden Ansprüche, wobei die Wirtszelle das Nukleinsäuremolekül genomisch stabil integriert enthält.

9. Wirtszelle nach einem der vorangehenden Ansprüche, wobei das Nukleinsäuremolekül dsDNA, ssDNA, PNA, CNA, RNA oder mRNA oder Kombinationen davon umfasst.

10. Wirtszelle nach einem der vorangehenden Ansprüche, wobei das Nukleinsäuremolekül in einer Expressionskassette oder einem Expressionsvektor umfasst ist.

11. Wirtszelle nach Anspruch 10, wobei die Expressionskassette Promotor- und Terminatorsequenzen umfasst, oder jeweils unterschiedliche Paare aus Promotor- und Terminatorsequenzen umfasst.

12. Verfahren zur Produktion von Bioethanol, umfassend die Expression eines Nukleinsäuremoleküls, wie in einem der Ansprüche 1 bis 10 definiert, in einer Wirtszelle nach einem der Ansprüche 1 bis 11.

13. Verwendung einer Wirtszelle nach einem der Ansprüche 1 bis 11 zur Produktion von Bioethanol oder zur rekombinanten Fermentation von Pentose-haltigem Biomaterial.

## Claims

1. A host cell containing a nucleic acid molecule comprising three nucleic acid sequences each of which coding for a polypeptide of a bacterial L-arabinose metabolic pathway, wherein the three nucleic acid sequences are araA (L-arabinose isomerase), araB (L-ribulokinase) and araD (L-ribulose-5-P-4-epimerase),
wherein the nucleic acid sequence for araA is derived from *Bacillus licheniformis* or *Clostridium acetobutylicum,*
and wherein the host cell is a fungus cell.

2. The host cell according to claim 1, which is a yeast cell, such as *Saccharomyces* species, *Kluyveromyces* sp., *Hansenula* sp., *Pichia* sp. or *Yarrowia* sp.

3. The host cell according to claim 1 or 2, wherein the nucleic acid sequence for araB and the nucleic acid sequence for araD originate from *E. coli.*

4. The host cell according to any one of claims 1 to 3, wherein the nucleic acid sequence for araA comprises a nucleic acid sequence with SEQ ID NO: 3, 4 or 5.

5. The host cell according to any one of the preceding claims, wherein at least two of the three nucleic acid sequences are codon-optimised for use in a host cell.

6. The host cell according to any one of the preceding claims, wherein the nucleic acid sequence for araB comprises a nucleic acid sequence with SEQ ID NO: 1,
and/or wherein the nucleic acid sequence for araD comprises a nucleic acid sequence with SEQ ID NO: 2.

7. The host cell according to any one of the preceding claims, wherein the nucleic acid molecule comprises the nucleic acid sequence with SEQ ID NO: 1, the nucleic acid sequence with SEQ ID NO: 2 and the nucleic acid sequence with SEQ ID NO: 3, 4 or 5.

8. The host cell according to any one of the preceding claims, wherein the host cell contains the nucleic acid molecule integrated in a genomically stable manner.

9. The host cell according to any one of the preceding claims, wherein the nucleic acid molecule comprises dsDNA, ssDNA, PNA, CNA, RNA or mRNA or combinations thereof.

10. The host cell according to any one of the preceding claims, wherein the nucleic acid molecule is comprised in an expression cassette or in an expression vector.

11. The host cell according to claim 10, wherein the expression cassette comprises promoter and terminator sequences, or comprises different pairs of promoter and terminator sequences, respectively.

12. A method for producing bioethanol, comprising the expression of a nucleic acid molecule, as defined in any one of claims 1 to 10, in a host cell according to any one of claims 1 to 11.

13. Use of a host cell according to any one of claims 1 to 11 for producing bioethanol or for recombinant fermentation of pentose-containing biomaterial.

## Revendications

1. Cellule hôte contenant une molécule d'acide nucléique qui comprend trois séquences d'acides nucléiques dont chacune code pour un polypeptide de la voie métabolique bactérienne du L-arabinose, les trois séquences d'acides nucléiques étant araA (L-arabinose-isomérase), araB (L-ribulokinase) et araD (L-ribulose-5-P-4-épimérase) ; la séquence d'acide nucléique pour araA dérivant de *Bacillus licheniformis* ou de *Clostridium acetobutylicum* ; et la cellule hôte étant une cellule fongique.

2. Cellule hôte selon la revendication 1, qui est une cellule de levure, telle que l'espèce *Saccharomyces*, *Kluyveromyces* sp., *Hansenula* sp., *Pichia* sp. ou *Yarrowia* sp.

3. Cellule hôte selon la revendication 1 ou 2, dans laquelle la séquence d'acide nucléique pour araB et la séquence d'acide nucléique pour araD dérivent de *E. coli.*

4. Cellule hôte selon l'une des revendications 1 à 3, dans laquelle la séquence d'acide nucléique pour araA comprend une séquence d'acide nucléique SEQ ID NO:3, 4 ou 5.

5. Cellule hôte selon l'une des revendications précédentes, dans laquelle au moins deux des trois séquences d'acides nucléiques sont à optimisation de codons pour une utilisation dans une cellule hôte.

6. Cellule hôte selon l'une des revendications précédentes, dans laquelle la séquence d'acide nucléique pour araB comprend une séquence d'acide nucléique SEQ ID NO:1, et/ou dans laquelle la séquence d'acide nucléique pour araB comprend une séquence d'acide nucléique SEQ ID NO:2.

7. Cellule hôte selon l'une des revendications précédentes, dans laquelle la molécule d'acide nucléique comprend la séquence d'acide nucléique SEQ ID NO:1, la séquence d'acide nucléique SEQ ID NO:2, ainsi que la séquence d'acide nucléique SEQ ID NO:3, 4 ou 5.

8. Cellule hôte selon l'une des revendications précédentes, la cellule hôte contenant, stablement intégrée au niveau génomique, la molécule d'acide nucléique.

9. Cellule hôte selon l'une des revendications précédentes, dans laquelle la molécule d'acide nucléique comprend de l'ADNdb, de l'ADNsb, du PNA, du CNA, de l'ARN ou de l'ARNm ou une combinaison de ces derniers.

10. Cellule hôte selon l'une des revendications précédentes, dans laquelle la molécule d'acide nucléique est comprise dans une cassette d'expression ou un vecteur d'expression.

11. Cellule hôte selon la revendication 10, dans laquelle la cassette d'expression comprend des séquences promotrices et terminatrices, ou des paires différentes de chacune des séquences promotrices et terminatrices.

12. Procédé de production de bioéthanol, comprenant l'expression d'une molécule d'acide nucléique telle que définie dans l'une quelconque des revendications 1 à 10, dans une cellule hôte selon l'une des revendications 1 à 11.

13. Utilisation d'une cellule hôte selon l'une des revendications 1 à 11 pour la production de bioéthanol ou pour la fermentation par recombinaison d'un biomatériau contenant du pentose.
